(19)

**Europäisches Patentamt**
**European Patent Office**
**Office européen des brevets**

(11) **EP 3 932 420 A1**

(12) **EUROPEAN PATENT APPLICATION**

(43) Date of publication:
**05.01.2022 Bulletin 2022/01**

(51) Int Cl.:
*A61K 38/48* *(2006.01)*    *A61P 31/00* *(2006.01)*
*A61P 31/04* *(2006.01)*    *A61P 31/10* *(2006.01)*
*A61P 31/12* *(2006.01)*    *A61P 11/00* *(2006.01)*

(21) Application number: **21180866.2**

(22) Date of filing: **29.01.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.01.2014 GB 201401480**
**31.03.2014 GB 201405784**

(62) Document number(s) of the earlier application(s) in
accordance with Art. 76 EPC:
**15708564.8 / 3 099 317**

(71) Applicant: **Enzymatica AB**
**223 70 Lund (SE)**

(72) Inventors:
• **BLOM,, Ulf Thomas**
 **S-24633 Löddeköpinge (SE)**
• **CLARSUND,, Mats Peter**
 **S-22651 Lund (SE)**

(74) Representative: **Potter Clarkson**
**The Belgrave Centre**
**Talbot Street**
**Nottingham NG1 5GG (GB)**

Remarks:
This application was filed on 22-06-2021 as a
divisional application to the application mentioned
under INID code 62.

(54) **NOVEL TREATMENTS**

(57) The present invention provides polypeptides having protease activity for use in the treatment or prevention of microbial infections in a subject with or susceptible to immunodeficiency. For example, the polypeptide may be administered as a mouth spray, nasal spray, lozenge, pastille, chewing gum or liquid to treat or prevent microbial infections in a patient with primary immunodeficiency. In particular, the polypeptides are useful in the treatment or prevention of rhinorrhea and/or fungal infection of the oral cavity and/or gum sores. In one embodiment, the polypeptide is a trypsin enzyme from Atlantic cod, or a fragment or variant thereof.

FIGURE 1

**Description**

**Field of Invention**

The present invention relates to polypeptide-based agents for use in the treatment or prevention of microbial infections in a subject with or susceptible to immunodeficiency.

**Background**

Primary immunodeficiencies (PIDs) are a diverse group of over 300 genetic disorders that fundamentally affect the development and/or functionality of the immune system. Most of them are rare monogenic disorders, but the spectrum of PIDs is constantly expanding with the identification of novel immunodeficiency syndromes through next generation sequencing technologies and improved clinical awareness. Patients classically present with a higher susceptibility to infections or infection with unusual organisms and may also develop autoimmunity or autoinflammatory disease and lymphoreticular malignancies. Although minimal or supportive therapies are effective for many of these conditions, the severest require definitive early treatment in order to prevent chronic morbidity and early mortality.

The incidence of most primary immunodeficiencies is uncertain because of the lack of a national registry or reporting by government health surveys. In the United States, as many as 500,000 persons have one of the known primary immunodeficiencies, with about 50,000 cases diagnosed each year. The primary immunodeficiencies appear to affect males and females about equally.

PIDs are also an un-addressed public health issue in Europe, with an estimated two million children and adults suffering from recurrent infections within the member states without being diagnosed and so not being offered treatment.

A number of different treatment strategies have been developed for the management of primary immunodeficiencies, including:

*(a) Intravenous immunoglobulin (ivlg)*

For the past 20 years, intravenously administered immune globulin (ivlg) has been used in the treatment of agammaglobulinemia. This agent is now standard therapy for most antibody deficiencies. Most commonly, IVIG is used in patients with X-linked agammaglobulinemia, common variable immunodeficiency, X-linked hyper IgM, severe combined immunodeficiency, Wiskott-Aldrich syndrome, and selective IgG class deficiency.

Ivlg is also used, or is being considered for use, in a wide variety of other illnesses. Consequently, its limited availability is a concern.

*(b) Bone marrow transplantation*

Bone marrow transplants from HLA-identical donors can be curative in patients with cellular immune deficiencies such as severe combined immunodeficiency, Wiskott-Aldrich syndrome, and DiGeorge syndrome, and may be beneficial in patients with chronic granulomatous disease. Bone marrow transplantation currently has no role in the treatment of antibody deficiencies.

HLA-identical donors are not always available. Long-term survival may be lower with bone marrow transplants from haploidentical donors. Thus, investigations of alternative strategies, such as gene therapy, could benefit the management of patients with primary immunodeficiency disorders who otherwise would require bone marrow transplantation.

*(c) Antibiotics and other therapies*

When recurrent infections are a problem, many patients with primary immunodeficiencies are managed with antibiotics alone or in combination with IVIG. For example, in patients with chronic granulomatous disease, prophylactic therapy with trimethoprim-sulfamethoxazole (Bactrim, Septra) reduces the incidence of severe infections by 50 percent. Similarly, treatment for complement deficiencies is directed at preventing infection, and consists of antibiotic prophylaxis and immunizations for encapsulated bacteria (e.g. heptovalent pneumococcal vaccine, Haemophilus b conjugate vaccine, meningococcal polysaccharide vaccine).

Other treatments for primary immunodeficiencies include enzyme replacement in patients with adenosine deaminase deficiency (a subtype of severe combined immunodeficiency) and cytokine therapy in patients with chronic granulomatous disease.

More recently, advances have also been made in gene therapy for primary immunodeficiencies (for example, see Rivat et al., 2012, Hum. Gene Ther. 23(7):668-675).

However, there remains a need for improved therapies for managing microbial infections in subjects with an immunodeficiency, such as a primary immunodeficiency or drug-induced immunodeficiency, in order to improve the quality of life for such patients.

**Summary of Invention**

The first aspect of the invention provides a polypeptide having protease activity for use in the treatment or prevention of microbial infections in a subject with or susceptible to immunodeficiency.

By "protease" we include any enzyme capable of catalysing proteolysis *in vivo,* in the mammalian (e.g. human) body. Thus, any type of protease may be utilised in the invention, including but not limited to serine proteases (such as trypsins/chymotrypsins), threonine proteases, cysteine proteases, aspartate proteases, glutamic acid proteases and metalloproteases.

By "immunodeficiency" we mean a condition in which the subject's immune disease is compromised, in whole or in part. The immunodeficiency may be acquired or secondary, e.g. following treatment with an immunosuppressive therapy, or may be primary, e.g. a naturally-occurring disorder in which part of the body's immune system is missing or does not function normally. Thus, in one embodiment the immunodeficiency is a secondary or acquired immunodeficiency.

For example, the immunodeficiency in the subject may arise from receiving treatment with an immunosuppressant therapy (such as glucocorticoids, cytostatics, antibodies, drugs acting on immunophilins, interferons, opioids, TNF-binding proteins, mycophenolate and radiation therapy).

Immunosuppressant therapies are commonly-used in medicine, for example:

(a) to prevent the rejection of transplanted organs and tissues (e.g. bone marrow, heart, kidney, liver);
(b) to treat autoimmune diseases or diseases that are of autoimmune origin (e.g. rheumatoid arthritis, multiple sclerosis, myasthenia gravis, systemic lupus erythematosus, sarcoidosis, focal segmental glomerulosclerosis, Crohn's disease, Behcet's Disease, pemphigus, and ulcerative colitis); and
(c) to treat other non-autoimmune inflammatory diseases (e.g. long term allergic asthma control).

In a further embodiment, the immunodeficiency is a naturally-occurring immunodeficiency. For example, the immunodeficiency may be due to a primary immunodeficiency (see below), a cancer (such as leukemia, lymphoma, multiple myeloma), chronic infection (such as acquired immunodeficiency syndrome or AIDS), malnutrition and/or aging.

Primary immunodeficiencies include a variety of disorders that render patients more susceptible to infections. If left untreated, these infections may be fatal. Common primary immunodeficiencies include disorders of humoral immunity (affecting B-cell differentiation or antibody production), T-cell defects and combined B- and T-cell defects, phagocytic disorders, and complement deficiencies. Major indications of these disorders include multiple infections despite aggressive treatment, infections with unusual or opportunistic organisms, failure to thrive or poor growth, and a positive family history. Early recognition and diagnosis can alter the course of primary immunodeficiencies significantly and have a positive effect on patient outcome.

In one embodiment, the patient has a primary immunodeficiency selected from the group consisting of the indications listed in Tables I to VIII.

**Table I**

Combined T and B-cell immunodeficiencies

In these disorders both T lymphocytes and B lymphocytes are dysfunctional or decreased in number. The main members are various types of severe combined immunodeficiency (SCID).

1. T-/B+ SCID (T cells predominantly absent): yc deficiency, JAK3 deficiency, interleukin 7 receptor chain α deficiency, CD45 deficiency, CD3δ/CD3ε deficiency.
2. T-/B- SCID (both T and B cells absent): RAG 1/2 deficiency, DCLRE1C deficiency, adenosine deaminase (ADA) deficiency, reticular dysgenesis
3. Omenn syndrome
4. DNA ligase type IV deficiency
5. Cernunnos deficiency
6. CD40 ligand deficiency
7. CD40 deficiency
8. Purine nucleoside phosphorylase (PNP) deficiency
9. CD3y deficiency
10. CD8 deficiency

11. ZAP-70 deficiency
12. Ca++ channel deficiency
13. MHC class I deficiency
14. MHC class II deficiency
15. Winged helix deficiency
16. CD25 deficiency
17. STAT5b deficiency
18. Itk deficiency
19. DOCK8 deficiency

**Table II**

Predominantly antibody deficiencies

In primary antibody deficiencies, one or more isotypes of immunoglobulin are decreased or fail to function properly.

1. Absent B cells with a resultant severe reduction of all types of antibody: X-linked agammaglobulinemia (btk deficiency, or Bruton's agammaglobulinemia), μ-Heavy chain deficiency, I 5 deficiency, Igα deficiency, BLNK deficiency, thymoma with immunodeficiency
2. B cells low but present or normal, but with reduction in 2 or more isotypes (usually IgG & IgA, sometimes IgM): common variable immunodeficiency (CVID), ICOS deficiency, CD19 deficiency, TACI (TNFRSF13B) deficiency, BAFF receptor deficiency.
3. Normal numbers of B cells with decreased IgG and IgA and increased IgM: Hyper-IgM syndromes
4. Normal numbers of B cells with isotype or light chain deficiencies: heavy chain deletions, kappa chain deficiency, isolated IgG subclass deficiency, IgA with IgG subclass deficiency, selective immunoglobulin A deficiency
5. Specific antibody deficiency to specific antigens with normal B cell and normal Ig concentrations
6. Transient hypogammaglobulinemia of infancy (THI)

**Table III**

Other well defined immunodeficiency syndrome

A number of syndromes escape formal classification but are otherwise recognisable by particular clinical or immunological features.

1. Wiskott-Aldrich syndrome
2. DNA repair defects not causing isolated SCID: ataxia telangiectasia, ataxia-like syndrome, Nijmegen breakage syndrome, Bloom syndrome
3. DiGeorge syndrome (when associated with thymic defects)
4. Various immuno-osseous dysplasias (abnormal development of the skeleton with immune problems): cartilage-hair hypoplasia, Schimke syndrome
5. Hermansky-Pudlak syndrome type 2
6. Hyper-IgE syndrome
7. Chronic mucocutaneous candidiasis
8. Hepatic venoocclusive disease with immunodeficiency (VODI)
9. XL-dyskeratosis congenita (Hoyeraal-Hreidarsson syndrome)

**Table IV**

Diseases of immune dysregulation

In certain conditions, the regulation rather than the intrinsic activity of parts of the immune system is the predominant problem.

1. Immunodeficiency with hypopigmentation or albinism: Chediak-Higashi syndrome, Griscelli syndrome type 2
2. Familial hemophagocytic lymphohistiocytosis: perforin deficiency, MUNC13D deficiency, syntaxin 11 deficiency
3. X-linked lymphoproliferative syndrome

4. Syndromes with autoimmunity:

(a) Autoimmune lymphoproliferative syndrome: type 1a (CD95 defects), type 1b (Fas ligand defects), type 2a (CASP10 defects), type 2b (CASP8 defects)
(b) APECED (autoimmune polyendocrinopathy with candidiasis and ectodermal dystrophy)
(c) IPEX (immunodysregulation polyendocrinopathy enteropathy X-linked syndrome)
(d) CD25 deficiency

**Table V**

Congenital defects of phagocyte number, function, or both

In certain conditions, either the number of phagocytes is reduced or their functional capacity is impaired.

1. Severe congenital neutropenia: due to ELA2 deficiency (with myelodysplasia)
2. Severe congenital neutropenia: due to GFI1 deficiency (with T/B lymphopenia)
3. Kostmann syndrome
4. Neutropenia with cardiac and urogenital malformations
5. Glycogen storage disease type 1b
6. Cyclic neutropenia
7. X-linked neutropenia/myelodysplasia
8. P14 deficiency
9. Leukocyte adhesion deficiency type 1
10. Leukocyte adhesion deficiency type 2
11. Leukocyte adhesion deficiency type 3
12. RAC2 deficiency (Neutrophil immunodeficiency syndrome)
13. Beta-actin deficiency
14. Localized juvenile periodontitis
15. Papillon-Lefèvre syndrome
16. Specific granule deficiency
17. Shwachman-Diamond syndrome
18. Chronic granulomatous disease: X-linked
19. Chronic granulomatous disease: autosomal (CYBA)
20. Chronic granulomatous disease: autosomal (NCF1)
21. Chronic granulomatous disease: autosomal (NCF2)
22. IL-12 and IL-23 $\beta$1 chain deficiency
23. IL-12p40 deficiency
24. Interferon $\gamma$ receptor 1 deficiency
25. Interferon $\gamma$ receptor 2 deficiency
26. STAT1 deficiency (2 forms)
27. AD hyper-IgE
28. AR hyper-IgE
29. Pulmonary alveolar proteinosis

**Table VI**

Defects in innate immunity

Several rare conditions are due to defects in the innate immune system. Many of these conditions are associated with skin problems.

1. Hypohidrotic ectodermal dysplasia

(a) NEMO deficiency
(b) IKBA deficiency

2. EDA-ID
3. IRAK-4 deficiency

4. MyD88 deficiency

5. WHIM syndrome (warts, hypogammaglobulinaemia, infections, myleokathexis)

6. Epidermodysplasia verruciformis

7. Herpes simplex encephalitis

8. Chronic mucocutaneous candidiasis

9. Trypanosomiasis

**Table VII**

Autoinflammatory disorder

Rather than predisposing for infections, most of the autoinflammatory disorders lead to excessive inflammation. Many manifest themselves as periodic fever syndromes.

1. Familial Mediterranean fever

2. TNF receptor associated periodic syndrome (TRAPS)

3. Hyper-IgD syndrome (HIDS)

4. *CIAS1*-related diseases:

    (a) Muckle-Wells syndrome

    (b) Familial cold autoinflammatory syndrome

    (c) Neonatal onset multisystem inflammatory disease

5. PAPA syndrome (pyogenic sterile arthritis, pyoderma gangrenosum, acne)

6. Blau syndrome

7. Chronic recurrent multifocal osteomyelitis and congenital dyserythropoietic anemia (Majeed syndrome)

8. DIRA (deficiency of the IL-1 receptor antagonist)

**Table VIII**

Complement deficiencies

Complement deficiencies predispose to infections but also to autoimmune conditions.

1. C1q deficiency (lupus-like syndrome, rheumatoid disease, infections)

2. C1r deficiency (idem)

3. C1s deficiency

4. C4 deficiency (idem)

5. C2 deficiency (lupus-like syndrome, vasculitis, polymyositis, pyogenic infections)

6. C3 deficiency (recurrent pyogenic infections)

7. C5 deficiency (Neisserial infections, SLE)

8. C6 deficiency (idem)

9. C7 deficiency (idem, vasculitis)

10. C8a deficiency

11. C8b deficiency

12. C9 deficiency (Neisserial infections)

13. C1-inhibitor deficiency (hereditary angioedema)

14. Factor I deficiency (pyogenic infections)

15. Factor H deficiency (haemolytic-uraemic syndrome, membranoproliferative glomerulonephritis)

16. Factor D deficiency (Neisserial infections)

17. Properdin deficiency (Neisserial infections)

18. MBP deficiency (pyogenic infections)

19. MASP2 deficiency

20. Complement receptor 3 (CR3) deficiency

21. Membrane cofactor protein (CD46) deficiency

22. Membrane attack complex inhibitor (CD59) deficiency

23. Paroxysmal nocturnal hemoglobinuria

24. Immunodeficiency associated with ficolin 3 deficiency

It will be appreciated by persons skilled in the art that the polypeptides of the invention do not provide a cure for primary immunodeficiencies *per se*. Rather, the polypeptides seek to alleviate or prevent one or more of the symptoms of microbial infections associated with such disorders.

Thus, by "treatment" we include the alleviation, in part or in whole, of the symptoms of microbial infections, including but not limited to bacterial, viral and fungal infections, in patients with a primary immunodeficiency.

By "prevention" we include the reduction in risk of microbial infection developing in patients with a primary immunodeficiency. However, it will be appreciated that such prevention may not be absolute, i.e. it may not prevent all such patients developing microbial infections. As such, the terms "prevention" and "prophylaxis" may be used interchangeably.

In one embodiment, the microbial infection is selected from the group consisting of bacterial infections, viral infections, fungal infections and yeast infections.

In particular, the polypeptides of the invention are for use in the treatment or prevention of secondary infections of the mouth and/or pharynx (e.g. oropharynx). For example, the polypeptides may be used in the treatment or prevention of rhinorrhea and/or fungal infection of the oral cavity and/or gum sores.

The polypeptides of the invention are particularly useful in the treatment or prevention of microbial infections in PI patients who suffer from regular episodes of infection (for example, at least five microbial infections a year, *e.g.* at least ten, fifteen, twenty, thirty or more microbial infections a year).

The polypeptides of the invention may exhibit trypsin activity. By "trypsin activity" we mean that the polypeptide exhibits a peptidase activity of a trypsin enzyme (EC 3,4,21,4) or of a related peptidase (such as chymotrypsin enzymes, EC 3,4,21,1).

The polypeptides of the invention may be naturally occurring or non-naturally occurring.

In one embodiment, the polypeptide is derived, directly or indirectly, from a fish, such as Atlantic cod (*Gadus morhua*), Atlantic and Pacific salmon (*e.g. Salmo salar* and species of *Oncorhynchus*) and Alaskan Pollock (*Theragra chalcogramma*).

Three major isozymes of trypsin have been characterised from Atlantic cod, designated Trypsin I, II and III (see Ásgeirsson et al., 1989, Eur. J. Biochem. 180:85-94, the disclosures of which are incorporated herein by reference). For example, see GenBank Accession No. ACO90397.

In addition, Atlantic cod expresses two major isozymes of chymotrypsin, designated Chymotrypsin A and B (see Ásgeirsson & Bjarnason, 1991, Comp. Biochem. Physiol. B 998:327-335, the disclosures of which are incorporated herein by reference). For example, see GenBank Accession No. CAA55242.1.

In one embodiment, the polypeptide comprises or consists of an amino acid sequence of trypsin I from Atlantic cod (*Gadus morhua*), *i.e.* SEQ ID NO: 1:

```
IVGGYECTKHSQAHQVSLNSGYHFCGGSLVSKDWVVSAAHCYKSVLRVRLGEHHIRVNEGTEQYI
SSSSVIRHPNYSSYNINNDIMLIKLTKPATLNQYVHAVALPTECAADATMCTVSGWGNTMSSVAD
GDKLQCLSLPILSHADCANSYPGMITQSMFCAGYLEGGKDSCQGDSGGPVVCNGVLQGVVSWGYG
CAERDHPGVYAKVCVLSGWVRDTMANY
```

**[SEQ ID NO: 1]**

or a fragment, variant, derivative or fusion thereof (or a fusion of said fragment, variant or derivative) which retains the trypsin activity of said amino acid sequence.

In a preferred embodiment, the polypeptide comprises or consists of an amino acid sequence according to SEQ ID NO: 1. Such a polypeptide may be purified from Atlantic cod, for example as described in Ásgeirsson et al., 1989, Eur. J. Biochem. 180:85-94 (the disclosures of which are incorporated herein by reference).

Suitable exemplary polypeptides of the invention, and methods for their production, are also described in European Patent No. 1 202 743 B (the disclosures of which are incorporated herein by reference).

The term 'amino acid' as used herein includes the standard twenty genetically-encoded amino acids and their corresponding stereoisomers in the 'D' form (as compared to the natural 'L' form), omega-amino acids and other naturally-occurring amino acids, unconventional amino acids (e.g., $\alpha,\alpha$-disubstituted amino acids, N-alkyl amino acids, *etc.*) and chemically derivatised amino acids (see below).

When an amino acid is being specifically enumerated, such as 'alanine' or 'Ala' or 'A', the term refers to both L-alanine and D-alanine unless explicitly stated otherwise. Other unconventional amino acids may also be suitable components for polypeptides of the present invention, as long as the desired functional property is retained by the polypeptide. For the peptides shown, each encoded amino acid residue, where appropriate, is represented by a single letter designation, corresponding to the trivial name of the conventional amino acid.

In accordance with convention, the amino acid sequences disclosed herein are provided in the N-terminus to C-terminus direction.

In one embodiment, the polypeptides of the invention comprise or consist of L-amino acids.

Where the polypeptide comprises an amino acid sequence according to SEQ ID NO: 1, it may comprise additional amino acids at its N- and/or C- terminus beyond those of SEQ ID NO: 1, for example, the polypeptide may comprise additional amino acids at its C-terminus. Likewise, where the polypeptide comprises a fragment, variant or derivative of an amino acid sequence according to SEQ ID NO: 1, it may comprise additional amino acids at its N- and/or C- terminus.

Skilled persons will appreciate that the polypeptide of the invention need not correspond to the full length, naturally occurring trypsin protein. Instead, the polypeptide may correspond to a fragment of such a wildtype trypsin, provided that said fragment retains (at least in part) the trypsin activity of the naturally occurring trypsin protein from which it is derived.

Trypsin activity may be determined using methods well known in the art. For example, trypsin assay kits are commercially available from Abcam, Cambridge, UK (see Cat No. ab102531) and other suppliers. In one embodiment, trypsin activity is measured using Cbz-Gly-Pro-Arg-p-nitroanilide (Cbz-GPR-pNA) as a substrate, yielding a specific activity of at least 10 U/mg, for example at least 50 U/mg or at least 100 U/mg (see EP 1 202 743 B).

Thus, in one embodiment the polypeptide comprises or consists of a fragment of the amino acid sequence according to SEQ ID NO: 1.

Thus, the polypeptide may comprise or consist of at least 15 contiguous amino acid of SEQ ID NO: 1, for example at least 16, 17, 18, 19, 20, 30, 40, 50, 60, 70, 80, 90, 100, 110, 120, 130, 140, 150, 160, 170, 180, 190, 200, 210, 220, 230 or 240 contiguous amino acid of SEQ ID NO: 1.

For example, the fragment may comprise or consist of amino acid residues 61 to 77 of SEQ ID NO:1.

Alternatively, or in addition, the fragment may comprise or consist of amino acid residues 225 to 241 of SEQ ID NO:1.

It will be appreciated by persons skilled in the art that the polypeptide of the invention may alternatively comprise or consist of a variant of the amino acid sequence according to SEQ ID NO: 1 (or fragment thereof). Such a variant may be a non-naturally occurring variant.

By 'variants' of the polypeptide we include insertions, deletions and substitutions, either conservative or non-conservative. In particular we include variants of the polypeptide where such changes retain, at least in part, the trypsin activity of the said polypeptide. Such variants may be made using the methods of protein engineering and site-directed mutagenesis well known in the art using the recombinant polynucleotides (see example, see Molecular Cloning: a Laboratory Manual, 3rd edition, Sambrook & Russell, 2000, Cold Spring Harbor Laboratory Press, which is incorporated herein by reference).

In one embodiment, the variant has an amino acid sequence which has at least 50% identity with the amino acid sequence according to SEQ ID NO: 1 or a fragment thereof, for example at least 55%, 60%, 65%, 70%, 75%, 80%, 90%, 95%, 96%, 97%, 98% or at least 99% identity.

The percent sequence identity between two polypeptides may be determined using suitable computer programs, for example the GAP program of the University of Wisconsin Genetic Computing Group and it will be appreciated that percent identity is calculated in relation to polypeptides whose sequences have been aligned optimally.

The alignment may alternatively be carried out using the Clustal W program (as described in Thompson et al., 1994, Nuc. Acid Res. 22:4673-4680, which is incorporated herein by reference).

The parameters used may be as follows:

Fast pairwise alignment parameters: K-tuple(word) size; 1, window size; 5, gap penalty; 3, number of top diagonals; 5. Scoring method: x percent.

Multiple alignment parameters: gap open penalty; 10, gap extension penalty; 0.05.

Scoring matrix: BLOSUM.

Alternatively, the BESTFIT program may be used to determine local sequence alignments.

In one embodiment, the polypeptide having protease activity is a variant of SEQ ID NO:1 comprising one or more mutated amino acids selected from the group consisting of amino acid positions:
E21, H25, H29, V47, K49, D50, L63, H71, H72, R74, N76, T79, Y82, S85, S87, S89, N98, I99, V121, M135, V138, M145, V148, D150, K154, L160, M175, S179, A183, L185, V212, Y217, P225, A229, V233, L234, V238, N240, Y241 and/or M242.

(wherein the amino acid sequence and numbering is according to Protein Data Bank [PDB] entry '2EEK!', with the initial isoleucine of SEQ ID NO: 1 being numbered as position 1-16).

Thus, the polypeptide having protease activity may be a variant of SEQ ID NO:1 comprising one or more amino acids mutations selected from the group consisting of:

E21T, H25Y, H29(Y/N), V47I, K49E, D50Q, L63I, H71D, H72N, R74(K/E), N76(T/L), T79(S/N), Y82F, S85A, S87(K/R), S89R, N98T, I99L, V121I, M135Q, V138I, M145(T/L/V/E/K), V148G, D150S, K154(T/V), L160(I/A), M175(K/Q), S179N, A183V, L185G, V212I, Y217(D/H/S), P225Y, A229V, V233N, L234Y, V238I, N240S, Y241N and/or M242I.

For example, the polypeptide having protease activity may comprise or consist of the amino acid sequence of SEQ ID NO:1 with one of the following defined mutations (or combinations thereof):

(a) N240S, Y241N, S87K ("EZA-002");
(b) K154T ("EZA-003");
(c) K154L ("EZA-004");
(d) K154V ("EZA-005");
(e) K154E ("EZA-006");
(f) N98T ("EZA-007");
(g) I99L ("EZA-008");
(h) L185G, P225Y ("EZA-009");
(i) V212I ("EZA-0010");
(j) Y217D, M175K ("EZA-011");
(k) Y217H ("EZA-012");
(l) Y217S ("EZA-013");
(m) A229V ("EZA-014");
(n) H25Y ("EZA-015");
(o) H25N ("EZA-016");
(p) H29Y ("EZA-017");
(q) H71D ("EZA-018");
(r) H72N ("EZA-019");
(s) R74K ("EZA-020");
(t) R74E ("EZA-021");
(u) N76T ("EZA-022");
(v) N76L, Y82F ("EZA-023");
(w) T79S ("EZA-0024");
(x) T79N ("EZA-025");
(y) K49E, D50Q ("EZA-026");
(z) S87R ("EZA-027");
(aa) E21T, H71D, D150S, K154V ("EZA-028");
(bb) S179N, V233N ("EZA-029");
(cc) M135Q ("EZA-030");
(dd) M145K, V148G ("EZA-031");
(ee) M175Q ("EZA-032");
(ff) L63I, S85A ("EZA-033");
(gg) L160I ("EZA-034");
(hh) V138I, L160A, A183V ("EZA-035");
(ii) V121I ("EZA-036");
(jj) V47I, V238I, M242I ("EZA-037");
(kk) V238I ("EZA-038"); and
(ll) L234Y ("EZA-039")

Likewise, the polypeptide having protease activity may comprise or consist of the amino acid of SEQ ID NO:1 with one of the following defined mutations (or combinations thereof):

(a) H25N, N76T
(b) H25N, H29Y
(c) H25N, M135Q
(d) H29Y, T79N, M135Q
(e) I99L, V121I, L160I, Y217H
(f) V121I, L160I
(g) H72N, R74E, S87K
(h) H25N, M135Q, Y217H
(i) T79N, V121I, V212I
(j) H29Y, N76T, I99L, M135Q

(k) K49E, D50Q, N76L, Y82F, S179N, V233N
(l) M145K, V148G, N76L, Y82F, S179N, V233N
(m) H25N, N76T, S87K, K154T
(n) H25Q
(o) H25D
(p) H25S
(q) K24E, H25N
(r) Y97N
(s) N100D
(t) A120S, A122S
(u) M135E
(v) V204Q, A122S
(w) T79D
(x) R74D
(y) K49E
(z) K49S,D50Q
(aa) D50Q
(bb) Q178D
(cc) S87R

In one preferred embodiment, the polypeptide having protease activity is a variant of the amino acid sequence of SEQ ID NO:1 which does not comprise histidine at position 25. For example, the polypeptide having protease activity may comprise or consist of the amino acid sequence of SEQ ID NO:2 (comprising an H25N mutation; see box in sequence below):

```
16
 I
IVGGYECTKNSQAHQVSLNSGYHFCGGSLVSKDWVVSAAHCYKSVLRVRLGEHHIRVNEG

79
 I
TEQYISSSSVIRHPNYSSYNINNDIMLIKLTKPATLNQYVHAVALPTECAADAMCTVSG

141
 I
WGNTMSSVADGDKLQCLSLPILSHADCANSYPGMITQSMFCAGYLEGGKDSCQGDSGGPV

200
 I
VCNGVLQGVVSWGYGCAERDHPGVYAKVCVLSGWVRDTMANY
```

[SEQ ID NO: 2]

In an alternative preferred embodiment, the polypeptide having protease activity is a variant of the amino acid sequence of SEQ ID NO:1 which does not comprise lysine at position 160.
For example, the polypeptide having protease activity may comprise or consist of the amino acid sequence of SEQ ID NO: 3 (comprising an L160I mutation; see box in sequence below):

16
|
IVGGYECTKHSQAHQVSLNSGYHFCGGSLVSKDWVVSAAHCYKSVLRVRLGEHHIRVNEG

79
|
TEQYISSSSVIRHPNYSSYNINNDIMLIKLTKPATLNQYVHAVALPTECAADAMCTVSG

141
|
WGNTMSSVADGDKLQCLS⬚IPILSHADCANSYPGMITQSMFCAGYLEGGKDSCQGDSGGPV

200
|
VCNGVLQGVVSWGYGCAERDHPGVYAKVCVLSGWVRDTMANY

[SEQ ID NO: 3]

It will be appreciated by persons skilled in the art that the above identified mutations (defined by reference to the amino acid sequence of trypsin I of Atlantic cod, SEQ ID NO:1) could also be made in trypsins from other species. For example, the specific mutations highlighted in SEQ ID NOS: 2 and 3 (H25N and L160I, respectively) could be made in the trypsin from Alaskan Pollock (for example see GenBank: BAH70476.3, wherein the amino acid sequence of the active trypsin commences at position 120, such that H25 corresponds to H29 in BAH70476.3, *etc*).

In an alternative embodiment, the polypeptide having protease activity comprises or consists of the amino acid sequence of a naturally-occurring serine protease. Thus, the polypeptide having serine protease activity may consist of the amino acid sequence of a naturally-occurring trypsin, of either eukaryotic or prokaryotic origin. Specifically included are cold-adapted trypsins, such as a trypsin from Atlantic cod (*Gadus morhua*), Atlantic and Pacific salmon (*e.g. Salmo salar* and species of *Oncorhynchus*) and Alaskan Pollock (*Theragra chalcogramma*).

In a further embodiment of the first aspect of the invention, the polypeptide comprises or consists of a fusion protein.

By 'fusion' of a polypeptide we include an amino acid sequence corresponding to SEQ ID NO: 1 (or a fragment or variant thereof) fused to any other polypeptide. For example, the said polypeptide may be fused to a polypeptide such as glutathione-S-transferase (GST) or protein A in order to facilitate purification of said polypeptide. Examples of such fusions are well known to those skilled in the art. Similarly, the said polypeptide may be fused to an oligo-histidine tag such as His6 or to an epitope recognised by an antibody such as the well-known Myc tag epitope. Fusions to any variant or derivative of said polypeptide are also included in the scope of the invention.

The fusion may comprise a further portion which confers a desirable feature on the said polypeptide of the invention; for example, the portion may be useful in augmenting or prolonging the therapeutic effect. For example, in one embodiment the fusion comprises human serum albumin or a similar protein.

Alternatively, the fused portion may be, for example, a biotin moiety, a radioactive moiety, a fluorescent moiety, for example a small fluorophore or a green fluorescent protein (GFP) fluorophore, as well known to those skilled in the art. The moiety may be an immunogenic tag, for example a Myc tag, as known to those skilled in the art or may be a lipophilic molecule or polypeptide domain that is capable of promoting cellular uptake of the polypeptide, as known to those skilled in the art.

In a further embodiment of the first aspect of the invention, the polypeptide comprises or consists of one or more amino acids that are modified or derivatised.

Chemical derivatives of one or more amino acids may be achieved by reaction with a functional side group. Such derivatised molecules include, for example, those molecules in which free amino groups have been derivatised to form amine hydrochlorides, p-toluene sulphonyl groups, carboxybenzoxy groups, *t*-butyloxycarbonyl groups, chloroacetyl groups or formyl groups. Free carboxyl groups may be derivatised to form salts, methyl and ethyl esters or other types of esters and hydrazides. Free hydroxyl groups may be derivatised to form O-acyl or O-alkyl derivatives. Also included as chemical derivatives are those peptides which contain naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine and ornithine for lysine. Derivatives also include peptides containing one or more additions or deletions as long as the requisite activity is maintained. Other included modifications are amidation, amino terminal acylation (e.g. acetylation or thioglycolic acid amidation), terminal carboxylamidation (*e.g.* with ammonia or methylamine), and the like terminal modifications.

It will be further appreciated by persons skilled in the art that peptidomimetic compounds may also be useful. Thus, by 'polypeptide' we include peptidomimetic compounds which are have an anti-inflammatory activity of the polypeptide of SEQ ID NO: 1. The term 'peptidomimetic' refers to a compound that mimics the conformation and desirable features of a particular peptide as a therapeutic agent.

For example, the polypeptides of the invention include not only molecules in which amino acid residues are joined by peptide (-CO-NH-) linkages but also molecules in which the peptide bond is reversed. Such retro-inverso peptidomimetics may be made using methods known in the art, for example such as those described in Meziere et al. (1997) J. Immunol. 159, 3230-3237, which is incorporated herein by reference. This approach involves making pseudopeptides containing changes involving the backbone, and not the orientation of side chains. Retro-inverse peptides, which contain NH-CO bonds instead of CO-NH peptide bonds, are much more resistant to proteolysis. Alternatively, the polypeptide of the invention may be a peptidomimetic compound wherein one or more of the amino acid residues are linked by a -y($CH_2$NH)- bond in place of the conventional amide linkage.

In a further alternative, the peptide bond may be dispensed with altogether provided that an appropriate linker moiety which retains the spacing between the carbon atoms of the amino acid residues is used; it may be advantageous for the linker moiety to have substantially the same charge distribution and substantially the same planarity as a peptide bond.

It will be appreciated that the polypeptide may conveniently be blocked at its N- or C-terminus so as to help reduce susceptibility to exoproteolytic digestion.

A variety of uncoded or modified amino acids such as $_D$-amino acids and N-methyl amino acids have also been used to modify polypeptides. In addition, a presumed bioactive conformation may be stabilised by a covalent modification, such as cyclisation or by incorporation of lactam or other types of bridges, for example see Veber et al., 1978, Proc. Natl. Acad. Sci. USA 75:2636 and Thursell et al., 1983, Biochem. Biophys. Res. Comm. 111:166, which are incorporated herein by reference.

In one preferred embodiment, however, the polypeptide of the invention comprises one or more amino acids modified or derivatised by PEGylation, amidation, esterification, acylation, acetylation and/or alkylation.

It will be appreciated by persons skilled in the art that the polypeptides of the invention may be of any suitable length. Preferably, the polypeptides are between 10 and 30 amino acids in length, for example between 10 and 20, 12 and 18, 12 and 16, or 15 and 20 amino acids in length. Alternatively, the polypeptide may be between 150 and 250 amino acids in length, for example between 200 and 250, 210 and 240, 220 and 230, or 220 and 225 amino acids in length.

In one embodiment, the polypeptide is linear.

In a further embodiment, the polypeptide is a recombinant polypeptide.

Advantageously, the polypeptide is provided in a form suitable for delivery to the mucosa of the mouth and/or pharynx (e.g. oropharynx). For example, the polypeptide may be provided in a mouth spray, nasal spray, lozenge, pastille, chewing gum or liquid.

A second, related aspect of the invention provides a polypeptide as defined above in the preparation of a medicament for the treatment or prevention of microbial infections in a subject with or susceptible to immunodeficiency.

Exemplary embodiments of the second aspect of the invention are described above in relation to the first aspect of the invention.

The polypeptides of the invention, as well as nucleic acid molecules, vectors and host cells for producing the same, may be made using methods well known in the art (for example, see Sambrook & Russell, 2000, Molecular Cloning, A Laboratory Manual, Third Edition, Cold Spring Harbor, New York, the relevant disclosures in which document are hereby incorporated by reference).

Alternatively, the polypeptides of the invention may be synthesised by known means, such as liquid phase and solid phase synthesis (for example, t-Boc solid-phase peptide synthesis and BOP-SPPS).

It will be appreciated by persons skilled in the art that the present invention also includes pharmaceutically acceptable acid or base addition salts of the above described polypeptides. The acids which are used to prepare the pharmaceutically acceptable acid addition salts of the aforementioned base compounds useful in this invention are those which form non-toxic acid addition salts, i.e. salts containing pharmacologically acceptable anions, such as the hydrochloride, hydrobromide, hydroiodide, nitrate, sulphate, bisulphate, phosphate, acid phosphate, acetate, lactate, citrate, acid citrate, tartrate, bitartrate, succinate, maleate, fumarate, gluconate, saccharate, benzoate, methanesulphonate, ethanesulphonate, benzenesulphonate, p-toluenesulphonate and pamoate [i.e. 1,1'-methylene-bis-(2-hydroxy-3 naphthoate)] salts, among others.

Pharmaceutically acceptable base addition salts may also be used to produce pharmaceutically acceptable salt forms of the polypeptides. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g. potassium and sodium) and alkaline earth metal cations (e.g. calcium and magnesium), ammonium or water-soluble amine addition salts such as N-methylglucamine-(meglumine), and the lower alkanolammonium and other base salts of pharmaceutically acceptable organic amines, among others.

It will be further appreciated that the polypeptides of the invention may be lyophilised for storage and reconstituted in a suitable carrier prior to use. Any suitable lyophilisation method (e.g. spray drying, cake drying) and/or reconstitution techniques can be employed. It will be appreciated by those skilled in the art that lyophilisation and reconstitution can lead to varying degrees of activity loss and that use levels may have to be adjusted upward to compensate. Preferably, the lyophilised (freeze dried) polypeptide loses no more than about 20%, or no more than about 25%, or no more than about 30%, or no more than about 35%, or no more than about 40%, or no more than about 45%, or no more than about 50% of its activity (prior to lyophilisation) when rehydrated.

The polypeptides of the invention are typically provided in the form of a therapeutic composition, in which the polypeptide is formulated together with a pharmaceutically acceptable buffer, diluent, carrier, adjuvant or excipient. Additional compounds may be included in the compositions, including, chelating agents such as EDTA, citrate, EGTA or glutathione. The antimicrobial/therapeutic compositions may be prepared in a manner known in the art that is sufficiently storage stable and suitable for administration to humans and animals. The therapeutic compositions may be lyophilised, e.g., through freeze drying, spray drying, spray cooling, or through use of particle formation from supercritical particle formation.

By "pharmaceutically acceptable" we mean a non-toxic material that does not decrease the effectiveness of the trypsin activity of the polypeptide of the invention. Such pharmaceutically acceptable buffers, carriers or excipients are well-known in the art (see Remington's Pharmaceutical Sciences, 18th edition, A.R Gennaro, Ed., Mack Publishing Company (1990) and handbook of Pharmaceutical Excipients, 3rd edition, A. Kibbe, Ed ., Pharmaceutical Press (2000), he disclosures of which are incorporated herein by reference).

The term "buffer" is intended to mean an aqueous solution containing an acid-base mixture with the purpose of stabilising pH. Examples of buffers are Trizma, Bicine, Tricine, MOPS, MOPSO, MOBS, Tris, Hepes, HEPBS, MES, phosphate, carbonate, acetate, citrate, glycolate, lactate, borate, ACES, ADA, tartrate, AMP, AMPD, AMPSO, BES, CABS, cacodylate, CHES, DIPSO, EPPS, ethanolamine, glycine, HEPPSO, imidazole, imidazolelactic acid, PIPES, SSC, SSPE, POPSO, TAPS, TABS, TAPSO and TES.

The term "diluent" is intended to mean an aqueous or non-aqueous solution with the purpose of diluting the peptide in the therapeutic preparation. The diluent may be one or more of saline, water, polyethylene glycol, propylene glycol, ethanol or oils (such as safflower oil, corn oil, peanut oil, cottonseed oil or sesame oil).

The term "adjuvant" is intended to mean any compound added to the formulation to increase the biological effect of the polypeptide of the invention. The adjuvant may be one or more of zinc, copper or silver salts with different anions, for example, but not limited to fluoride, chloride, bromide, iodide, tiocyanate, sulfite, hydroxide, phosphate, carbonate, lactate, glycolate, citrate, borate, tartrate, and acetates of different acyl composition. The adjuvant may also be cationic polymers such as cationic cellulose ethers, cationic cellulose esters, deacetylated hyaluronic acid, chitosan, cationic dendrimers, cationic synthetic polymers such as poly(vinyl imidazole), and cationic polypeptides such as polyhistidine, polylysine, polyarginine, and peptides containing these amino acids.

The excipient may be one or more of carbohydrates, polymers, lipids and minerals. Examples of carbohydrates include lactose, glucose, sucrose, mannitol, and cyclodextrines, which are added to the composition, e.g., for facilitating lyophilisation. Examples of polymers are starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone, all of different molecular weight, which are added to the composition, e.g., for viscosity control, for achieving bioadhesion, or for protecting the lipid from chemical and proteolytic degradation. Examples of lipids are fatty acids, phospholipids, mono-, di-, and triglycerides, ceramides, sphingolipids and glycolipids, all of different acyl chain length and saturation, egg lecithin, soy lecithin, hydrogenated egg and soy lecithin, which are added to the composition for reasons similar to those for polymers. Examples of minerals are talc, magnesium oxide, zinc oxide and titanium oxide, which are added to the composition to obtain benefits such as reduction of liquid accumulation or advantageous pigment properties.

In one embodiment, the polypeptide may be provided together with a stabiliser, such as calcium chloride.

The polypeptides of the invention may be formulated into any type of therapeutic composition known in the art to be suitable for the delivery of polypeptide agents.

In one embodiment, the polypeptides may simply be dissolved in water, saline, polyethylene glycol, propylene glycol, ethanol or oils (such as safflower oil, corn oil, peanut oil, cottonseed oil or sesame oil), tragacanth gum, and/or various buffers. For example, where the polypeptide is formulated to oral administration (such as in a mouth spray), the therapeutic composition may comprise the polypeptide dissolved in water, glycerol and menthol. An exemplary mouth spray formulation is marketed within Scandinavia as *ColdZyme*® (by Enzymatica AB, Lund, Sweden).

In a preferred embodiment, the invention provides a protease polypeptide as described above in an osmotically active solution. For example, the polypeptide may be formulated in glycerol or glycerine. Without wishing to be bound by theory, it is believed that such osmotically active solutions facilitate movement of fluid from within microbial cells to the extracellular milieu. This, in turn, is believed to facilitate the therapeutic effect of the polypeptides of the invention by creating a thin, active barrier that inhibits (at least, in part) the uptake of microbial cells such as bacteria and viruses by the host epithelial cells, e.g. of the pharynx.

In a further embodiment, the therapeutic compositions of the invention may be in the form of a liposome, in which the polypeptide is combined, in addition to other pharmaceutically acceptable carriers, with amphipatic agents such as lipids, which exist in aggregated forms as micelles, insoluble monolayers and liquid crystals. Suitable lipids for liposomal formulation include, without limitation, monoglycerides, diglycerides, sulfatides, lysolecithin, phospholipids, saponin, bile acids, and the like. Suitable lipids also include the lipids above modified by poly(ethylene glycol) in the polar headgroup for prolonging bloodstream circulation time. Preparation of such liposomal formulations is can be found in for example US 4,235,871, the disclosures of which are incorporated herein by reference.

The therapeutic compositions of the invention may also be in the form of biodegradable microspheres. Aliphatic polyesters, such as poly(lactic acid) (PLA), poly(glycolic acid) (PGA), copolymers of PLA and PGA (PLGA) or poly(caprolactone) (PCL), and polyanhydrides have been widely used as biodegradable polymers in the production of microspheres. Preparations of such microspheres can be found in US 5,851,451 and in EP 0 213 303, the disclosures of which are incorporated herein by reference.

In a further embodiment, the therapeutic compositions of the invention are provided in the form of polymer gels, where polymers such as starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polyvinyl imidazole, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone are used for thickening of the solution containing the peptide. The polymers may also comprise gelatin or collagen.

It will be appreciated that the therapeutic compositions of the invention may include ions and a defined pH for potentiation of action of the polypeptides. Additionally, the compositions may be subjected to conventional therapeutic operations such as sterilisation and/or may contain conventional adjuvants such as preservatives, stabilisers, wetting agents, emulsifiers, buffers, fillers, *etc*.

In one preferred embodiment, the therapeutic composition comprises the polypeptide in a Tris or phosphate buffer, together with one or more of EDTA, xylitol, sorbitol, propylene glycol and glycerol.

A particularly preferred therapeutic composition of the invention is described in Example A below.

The therapeutic compositions according to the invention may be administered via any suitable route known to those skilled in the art. Thus, possible routes of administration include oral, buccal, parenteral (intravenous, subcutaneous, and intramuscular), topical, ocular, nasal, pulmonar, parenteral, vaginal and rectal. Also administration from implants is possible.

In one preferred embodiment, the therapeutic compositions are administered orally. For example, the polypeptide may be formulated as a mouth spray, nasal spray, lozenge, pastille, chewing gum, or conventional liquid for oral administration.

In an alternative embodiment, the therapeutic compositions are administered parenterally, for example, intravenously, intracerebroventricularly, intraarticularly, intra-arterially, intraperitoneally, intrathecally, intraventricularly, intrasternally, intracranially, intramuscularly or subcutaneously, or they may be administered by infusion techniques. They are conveniently used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Alternatively, the therapeutic compositions may be administered intranasally or by inhalation (for example, in the form of an aerosol spray presentation from a pressurised container, pump, spray or nebuliser with the use of a suitable propellant, such as dichlorodifluoromethane, trichlorofluoro-methane, dichlorotetrafluoro-ethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas). In the case of a pressurised aerosol, the dosage unit may be determined by providing a valve to deliver a metered amount. The pressurised container, pump, spray or nebuliser may contain a solution or suspension of the active polypeptide, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

The therapeutic compositions will be administered to a patient in a pharmaceutically effective dose. A 'therapeutically effective amount', or 'effective amount', or 'therapeutically effective', as used herein, refers to that amount which provides a therapeutic effect for a given condition and administration regimen. This is a predetermined quantity of active material calculated to produce a desired therapeutic effect in association with the required additive and diluent, i.e. a carrier or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, *etc*., as is well known in the art. The administration of the pharmaceutically effective dose can be carried out both by single administration in the form of an individual dose unit or else several smaller dose units and also by multiple administrations of subdivided doses at specific intervals. Alternatively, the dose may be provided as a continuous infusion over a prolonged period.

The polypeptides can be formulated at various concentrations, depending on the efficacy/toxicity of the compound being used. Preferably, the formulation comprises the active agent at a concentration of between 0.1 $\mu$M and 1 mM, more preferably between 1 $\mu$M and 500 $\mu$M, between 500 $\mu$M and 1 mM, between 300 $\mu$M and 700 $\mu$M, between 1 $\mu$M and 100 $\mu$M, between 100 $\mu$M and 200 $\mu$M, between 200 $\mu$M and 300 $\mu$M, between 300 $\mu$M and 400 $\mu$M, between 400 $\mu$M and 500 $\mu$M and most preferably about 500 $\mu$M.

Thus, the therapeutic formulation may comprise an amount of a polypeptide, or fragment, variant, fusion or derivative thereof, sufficient to kill or slow the growth of microorganisms, such as viruses, bacteria and yeasts, within the mouth and/or pharynx (*e.g.* oropharynx).

A third aspect of the invention provides method for the treatment or prevention of microbial infections in a subject with or susceptible to immunodeficiency, the method comprising administering to the subject a therapeutically-effective amount of a polypeptide as defined above in relation to the first aspect of the invention.

Exemplary embodiments of the third aspect of the invention are described above in relation to the first aspect of the invention.

In one embodiment, the microbial infection is selected from the group consisting of bacterial infections, viral infections, fungal infections and yeast infections.

In one embodiment, the polypeptides of the invention are for use in the treatment or prevention of secondary infections of the mouth and/or pharynx (e.g. oropharynx).

For example, the polypeptides may be used in the treatment or prevention of rhinorrhea and/or fungal infection of the oral cavity and/or gum sores.

Such microbial infections may conveniently be treated/prevented by administering a polypeptide of the invention in the form of a mouth spray, nasal spray, lozenge or the like. Such formulations allow the polypeptide of the invention to be exposed to the mucosal membranes of the mouth and/or pharynx (*e.g.* oropharynx) for a prolonged period, whereupon the trypsin activity of the polypeptide is exposed to infiltrating microorganisms.

Typically, the polypeptide of the invention will be administered repeatedly over a period of days, weeks or longer.

It will be appreciated by persons skilled in the art that the polypeptides of the present invention may be for use in combination with one or more additional therapeutic agents.

For example, the polypeptides of the present invention may be for use in combination with:

(a) conventional antibiotic agents (such as cephalosporins, tetracyclines, aminoglycosides and penicillins);
(b) antiviral agents (such as oseltamivir and zanamivir); and/or
(c) antifungal agents (such as nystatin, clortrimazole and flucanozole)

Additionally, the polypeptides of the present invention may be for use in combination with 'over-the-counter' cold and 'flu remedies, for example analgesics such as paracetamol and ibuprofen, and decongestants such as phenylephrine.

Persons skilled in the art will further appreciate that the uses and methods of the present invention have utility in both the medical and veterinary fields. Thus, the polypeptide medicaments may be used in the treatment of both human and non-human animals (such as horses, dogs and cats).

Preferably, however, the patient is human.

Preferred, non-limiting examples which embody certain aspects of the invention will now be described.

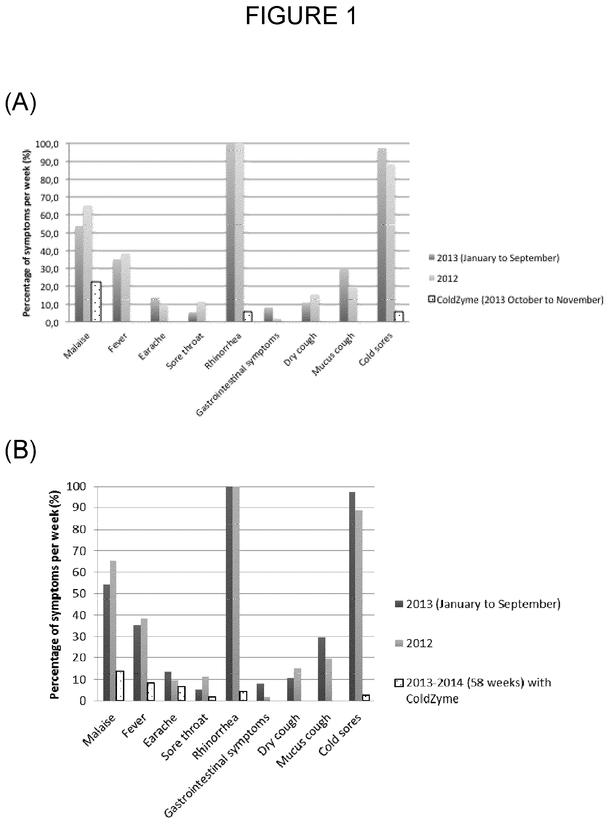

**Figure 1.** Percentage of various infection symptoms per week for a12 year old male patient diagnosed with CVID and treated weekly with subcutaneous injections of *Hizentra*® (human IgG). Baseline data compiled during 2012 and from January to September of 2013. *ColdZyme*® treatment was maintained for 9 weeks from October to November of 2013.

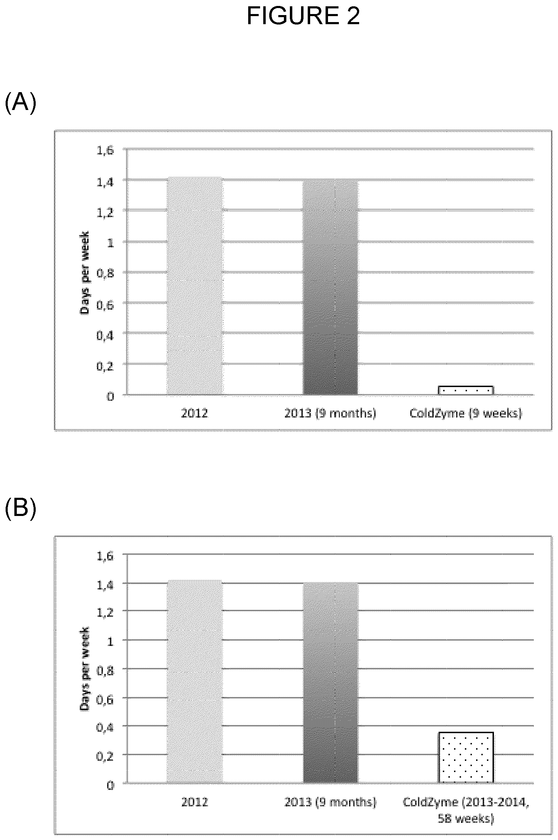

**Figure 2.** Average days per week spent at home from school for a 12-year old male patient diagnosed with CVID and treated weekly with subcutaneous injections of *Hizentra*® (IgG). Baseline data compiled during 2012 and from January to September of 2013. *ColdZyme*® treatment was maintained for 9 weeks from October to November of 2013.

## EXAMPLES

### Example A: Exemplary therapeutic formulation

An exemplary stock solution of a polypeptide of the invention, yrypsin I from Atlantic cod (SEQ ID NO:1), may be formulated as shown in Table 1:

**Table 1**

| Item description | Quantity |
|---|---|
| Purified water | 50 L |
| Glycerol | 50 L |
| Tris buffer stock solution | 1 L |
| Trypsin I from Atlantic cod | 300 000 U |

The pH is adjusted to 7.5.

A suitable therapeutic composition of trypsin I from Atlantic cod (SEQ ID NO:1) is also available commercially as *ColdZyme*® (Enzymatica AB, Lund, Sweden).

### Example B: Case study

| | |
|---|---|
| Patient: | 12-year old male |
| Diagnosis: | CVID (Common variable immunodeficiency) |
| Treatment history: | *Hizentra*®, subcutaneous immunoglobulin (Human) Amoxicillin, oral |
| Treatment: | *ColdZyme*®, 1U per dose, 2 doses per day |

Since 2003, the subject had received weekly subcutaneous injections of human immunoglobulin (*Hizentra*®, 4g weekly). However, prior to treatment with the polypeptide of the invention in late 2013, the subject suffered recurrent microbial infections of the ears, sinuses, nose, bronchi and lungs. The subject frequently exhibited continuous rhinorrhoea, fungal growth in the oral cavity and gingivitis with wounds in gum. As a consequence, the subject's quality of life had been severally compromised and he usually needed to stay at home from school at least one day every week. The month of November was often particularly challenging month for the subject since the recurrent infections often developed into pneumonia.

A period of prophylactic treatment with amoxicillin from August 2012 to May 2013 had little effect on the subject's recurrent symptoms.

The subject commenced twice daily treatment (morning and evening) with *ColdZyme*® mouth spray in October 2013; weekly administration of *Hizentra*® was continued throughout this period.

Within just three days of commencement of *ColdZyme*® treatment, the subject experienced a marked improvement in symptoms and quality of life (see Table 2):

**Table 2**

| Symptom | Before ColdZyme® | After three days treatment with ColdZyme® |
|---|---|---|
| Rhinorrhoea | Continuous | No Rhinorrhoea |
| Stuffed nose | Not possible to breathe through nose | Breathing through nose |
| Oral fungal infection | High | Very low |
| Wounds in gum tissue | High | Very low (healed for the first time on several years) |

The effect of treatment with *ColdZyme®* can also be seen clearly from Figures 1 and 2.

Figure 1(A) shows the percentage of various infection symptoms per week experienced by the subject during three time periods:

(a) During the whole of 2012 (treatment = *Hizentra®* and amoxicillin);
(b) From January to September 2013 (treatment = *Hizentra®* and amoxicillin); and
(c) From October 2013 to November 2013 (treatment = *Hizentra®* and *ColdZyme®*).

The dramatic reduction in all the observed infection symptoms during the period of treatment with *ColdZyme®* is immediately evident.

Figure 1(B) shows the percentage of various infection symptoms per week experienced by the same subject during an extended study period of 58 weeks

Figure 2(A) shows the average number of days per week the subject was absent from school due to the severity of infection symptoms during the same three time periods in Figure 1. The dramatic improvement following commencement of treatment with *ColdZyme®* is again immediately evident.

Such a quick onset of action and near total eradication of infection symptoms in the subject by treatment with the polypeptide of the invention was wholly unexpected, particularly given that the initial treatment period (October - November) coincided with the time of year during which the subject typically experienced the most severe infections. Understandably, both the subject and his mother were elated at the improvement in quality of life following over ten years of debilitating recurrent infections.

Figure 2(B) shows the average number of days per week the subject was absent from school due to the severity of infection symptoms during the same extended study period in Figure 1(B).

*Example C - Production of recombinant serine protease polypeptides*

Cloning

A synthesized gene encoding the serine protease polypeptide of interest was cloned into *E. coli* expression E3 vector (GenScript) without any tag.

Nucleic acid encoding wildtype trypsin I from Atlantic cod is shown below in SEQ ID NO: 4 (in pUC57)

```
  1   GAAGAAGATA AAATCGTTGG CGGCTATGAA TGCACGAAAC ACTCGCAGGC ACACCAGGTC
 61   TCACTGAACA GCGGTTACCA CTTTTGCGGC GGTAGTCTGG TTAGCAAAGA TTGGGTTGTT
121   AGTGCGGCCC ATTGCTATAA AAGCGTGCTG CGTGTTCGCC TGGGCGAACA TCACATTCGT
181   GTGAATGAAG GCACCGAACA GTACATTAGC TCTAGTAGCG TTATCCGCCA TCCGAACTAC
241   TCTAGTTACA ACATCAACAA CGATATCATG CTGATCAAAC TGACCAAACC GGCGACGCTG
301   AACCAGTATG TGCACGCCGT TGCACTGCCG ACCGAATGCG CAGCGGATGC AACCATGTGT
361   ACCGTGAGCG GCTGGGGTAA TACGATGAGC TCTGTTGCGG ATGGCGATAA ACTGCAGTGC
421   CTGTCTCTGC CGATTCTGAG TCATGCGGAT TGTGCCAACT CTTATCCGGG CATGATCACG
481   CAGAGCATGT TTTGCGCCGG TTACCTGGAA GGCGGTAAAG ATAGCTGCCA GGGTGATTCT
541   GGCGGTCCGG TGGTTTGTAA CGGCGTTCTG CAGGGTGTGG TTAGCTGGGG CTACGGTTGT
601   GCAGAACGTG ATCACCCGGG TGTCTATGCT AAAGTCTGTG TGCTGTCGGG CTGGGTCCGT
661   GATACGATGG CGAACTAT
```

**[SEQ ID NO: 4]**

A number of nucleic acid molecules encoding mutated versions of trypsin I from Atlantic cod were synthesised by conventional techniques, i.e. directed mutagenesis by PCR.

Refolding and Purification of trypsin

*Chemically competent E. coli* BL21 (DE3) cells were transformed with the E3 vector containing the nucleotide sequence encoding the serine protease polypeptide (trypsin) of interest using standard procedures, i.e. heat shock transformation.

The zymogen polypeptide (trypsinogen) was overexpressed and formed inclusion bodies in the cytoplasm of the host cells.

The cells after induction were harvested and lysed by sonication. After centrifugation, inclusion bodies were washed in buffer (50mM Tris, 10mM EDTA, 2% Triton X-100, 300mM NaCl, 2mM DTT, pH8.0) and dissolved in 50mM Tris, 8M Urea, pH8.0 and then dialyzed into 1× PBS, 10% Glycerol,pH7.4 at 4°C overnight.

The purity of the expressed zymogen polypeptide from refolding exhibited >90% purity and no other purification was deemed necessary.

The recombinant zymogen polypeptide was then activated by adding wildtype trypsin I purified from Atlantic cod (0.2 U/ml) and incubating at room temperature for 24 hours (see Example D).

Exemplary polypeptides

The following polypeptides were obtained or produced:

(a) Wildtype trypsin I purified from Atlantic cod ("WT-Tryp" or "wildtype");

(b) Recombinantly expressed wildtype trypsin I of Atlantic cod (SEQ ID NO:1, "R-Tryp"); and

(c) Thirty-eight different mutated versions of trypsin I of Atlantic cod (i.e. mutated sequences of SEQ ID NO:1).

The sequence mutations of the thirty-eight different mutated versions of cod trypsin I are shown in Table 3 below.

**Table 3**

| Sequences of exemplary trypsin polypeptides | |
|---|---|
| *Polypeptide name* | *Mutations relative to SEQ ID NO:1\** |
| EZA-001 | (none) |
| EZA-002 | N240S, Y241N, S87K |
| EZA-003 | K154T |
| EZA-004 | K154L |
| EZA-005 | K154V |
| EZA-006 | K154E |
| EZA-007 | N98T |
| EZA-008 | I99L |
| EZA-009 | L185G, P225Y |
| EZA-010 | V212I |
| EZA-011 | Y217D, M175K |
| EZA-012 | Y217H |
| EZA-013 | Y217S |
| EZA-014 | A229V |
| EZA-015 | H25Y |
| EZA-016 | H25N |
| EZA-017 | H29Y |
| EZA-018 | H71D |
| EZA-019 | H72N |
| EZA-020 | R74K |
| EZA-021 | R74E |
| EZA-022 | N76T |
| EZA-023 | N76L, Y82F |
| EZA-024 | T79S |
| EZA-025 | T79N |
| EZA-026 | K49E, D50Q |

(continued)

| Sequences of exemplary trypsin polypeptides | |
|---|---|
| *Polypeptide name* | *Mutations relative to SEQ ID NO:1\** |
| EZA-027 | S87R |
| EZA-028 | E21T, H71D, D150S, K154V |
| EZA-029 | S179N,V233N |
| EZA-030 | M135Q |
| EZA-031 | M145K, V148G |
| EZA-032 | M175Q |
| EZA-033 | L63I, S85A |
| EZA-034 | L160I |
| EZA-035 | V138I, L160A, A183V |
| EZA-036 | V121I |
| EZA-037 | V47I, V238I, M242I |
| EZA-038 | V238I |
| EZA-039 | L234Y |

*\* the amino acid numbering is according to Protein Data Bank (PDB) entry '2EEK!'*

The exemplary trypsin polypeptides were initially expressed as a zymogen polypeptide with the activation peptide MEEDK (SEQ ID NO: 5) fused to the N-terminus.

## Example D: Stability of wildtype and mutant forms of trypsin I of Atlantic cod, expressed recombinantly

This example summarizes the results from the activation of 39 recombinant trypsin mutants expressed in *E. coli.* The activity of the recombinant trypsin polypeptides (R-Tryp) was activated by wildtype trypsin I purified from Atlantic cod (WT-Tryp) after a 24 hours incubation.

Materials & Methods

*Expression of recombinant trypsins*

See Example C

*Assessment of stability*

The experiment designed for the activation and stability analysis of the recombinant samples was performed as follows:

Day 1: Activation of recombinant trypsin

Recombinant enzymes (0.2 U/ml) were activated by wild type trypsin (0.2 U/ml) at room temperature during 24 hours in a microtiter plate. The samples were mixed with 20mM Tris-HCl, 1mM $CaCl_2$, 50% glycerol, pH 7.6 to a final volume of 200$\mu$l.

Day 2: Activity and stability measurements

The activated recombinant enzymes were transferred to a new microtiter plate (II) and kept on ice to keep the enzymes stable and stop the activation process.

(a) Determination of initial activity A0

The activity of the activated enzyme (A0) was determined in a new microtiter plate (III) by mixing 245 $\mu$l of Gly-Pro-Arg in assay buffer, with 5 $\mu$l of recombinant enzyme from microtiter plate (II). The absorbance at 410 nm was followed and the activity was calculated according to the following formula:

$$U/ml = \mu mol/L.s = \frac{Slope_{410nm} * df * 60 * 10^3}{\varepsilon * l *} \quad (1)$$

where slope is the slope of the linear regression from the kinetic measurement of the trypsin activity at 30°C during 200 seconds; df is the dilution factor, 60 is the conversion of seconds to minutes, $\varepsilon$ is the extension coefficient equal to 8800 $M^{-1}$ $cm^{-1}$, l is the length of the light path equal to 0.7109cm, $10^3$ is the conversion mol/l to $\mu$mol/ml.

(b) Temperature inactivation

100 $\mu$l of the activated enzyme was transferred from microtiter plate (II) to a new microtiter plate (IV) and diluted to 200 $\mu$l to a final concentration of 50% glycerol, pH 7.6. Plate (IV) was incubated at 60°C for 3.5 hours (WT-Tryp loses 90% of the initial activity). The remaining activity was determined as under (a).

Day 3: Autocatalysis

100 $\mu$l of the activated enzyme was transferred from microtiter plate (II) to a new microtiter plate (V) containing 100$\mu$l of 0.1 U/ml trypsin in 25% glycerol and assay buffer, pH 7.6. The plate was incubated at 25°C for 8 hours (WT-Tryp loses 90% of the initial activity). The activity ($A_A X$) was determined as described under (a).

Results

Activity, thermostability and autocatalysis of thirty-nine exemplary serine protease polypeptides is reported in table 4 (recombinant wildtype cod trypsin, EZA-001, and thirty-eight mutants thereof). There is a considerable difference in activity among the mutants. Several mutants expressed improved temperature stability in comparison to wildtype trypsin that only had 5% remaining activity and several mutants showed substantially improved autocatalytic stabilities in comparison to wildtype trypsin.

## Table 4

Activity of 39 exemplary serine protease polypeptides

| Sample ID | Initial activity A0 (U/mg) | Thermostability: Remaining activity after inactivation at 60 ° C, Ax (U/ml) | Autocatalytic stability: Remaining activity after inactivation at 25° C, Ac (U/ml) | Relative thermostability (Ax/A0) | Relative autocatalytic stability (Ac/A0) |
|---|---|---|---|---|---|
| EZA001 | 0,52 | 0,10 | 0,07 | 0,20 | 0,13 |
| EZA002 | 0,48 | 0,05 | 0,01 | 0,11 | 0,02 |
| EZA003 | 0,52 | 0,09 | 0,05 | 0,18 | 0,09 |
| EZA004 | 0,48 | 0,09 | 0,04 | 0,19 | 0,07 |
| EZA005 | 0,36 | 0,07 | 0,02 | 0,19 | 0,06 |
| EZA006 | 0,39 | 0,10 | 0,03 | 0,26 | 0,07 |
| EZA007 | 0,30 | 0,10 | 0,01 | 0,33 | 0,04 |
| EZA008 | 0,36 | 0,11 | 0,09 | 0,31 | 0,26 |
| EZA009 | 0,35 | 0,06 | 0,03 | 0,16 | 0,09 |
| EZA010 | 0,44 | 0,12 | 0,12 | 0,28 | 0,28 |
| EZA011 | 0,36 | 0,10 | 0,11 | 0,28 | 0,31 |
| EZA012 | 0,35 | 0,13 | 0,11 | 0,37 | 0,31 |
| EZA013 | 0,36 | 0,07 | 0,05 | 0,20 | 0,13 |
| EZA014 | 0,41 | 0,10 | 0,07 | 0,25 | 0,17 |
| EZA015 | 0,39 | 0,09 | 0,11 | 0,24 | 0,27 |
| EZA016 | 0,36 | 0,22 | 0,21 | 0,60 | 0,56 |
| EZA017 | 0,35 | 0,14 | 0,15 | 0,41 | 0,42 |
| EZA018 | 0,39 | 0,05 | 0,02 | 0,13 | 0,04 |
| EZA019 | 0,37 | 0,10 | 0,10 | 0,27 | 0,27 |
| EZA020 | 0,39 | 0,07 | 0,03 | 0,19 | 0,08 |
| EZA021 | 0,38 | 0,11 | 0,09 | 0,30 | 0,23 |
| EZA022 | 0,49 | 0,09 | 0,17 | 0,18 | 0,34 |
| EZA023 | 0,39 | 0,18 | 0,16 | 0,47 | 0,41 |
| EZA024 | 0,43 | 0,09 | 0,07 | 0,21 | 0,17 |
| EZA025 | 0,39 | 0,17 | 0,14 | 0,43 | 0,37 |
| EZA026 | 0,33 | 0,15 | 0,15 | 0,44 | 0,46 |

| Sample ID | Initial activity A0 (U/mg) | Thermostability: Remaining activity after inactivation at 60 ° C, Ax (U/ml) | Autocatalytic stability: Remaining activity after inactivation at 25° C, Ac (U/ml) | Relative thermostability (Ax/A0) | Relative autocatalytic stability (Ac/A0) |
|---|---|---|---|---|---|
| EZA027 | 0,34 | 0,10 | 0,06 | 0,30 | 0,17 |
| EZA028 | 0,35 | 0,16 | 0,18 | 0,45 | 0,51 |
| EZA029 | 0,35 | 0,16 | 0,16 | 0,46 | 0,45 |
| EZA030 | 0,33 | 0,16 | 0,11 | 0,50 | 0,33 |
| EZA031 | 0,40 | 0,14 | 0,15 | 0,35 | 0,36 |
| EZA032 | 0,44 | 0,07 | 0,02 | 0,16 | 0,03 |
| EZA033 | 0,42 | 0,12 | 0,10 | 0,27 | 0,24 |
| EZA034 | 0,41 | 0,13 | 0,11 | 0,31 | 0,27 |
| EZA035 | 0,42 | 0,12 | 0,16 | 0,29 | 0,38 |
| EZA036 | 0,38 | 0,11 | 0,13 | 0,30 | 0,34 |
| EZA037 | 0,36 | 0,10 | 0,16 | 0,27 | 0,44 |
| EZA038 | 0,41 | 0,08 | 0,05 | 0,20 | 0,12 |
| EZA039 | 0,38 | 0,10 | 0,04 | 0,26 | 0,11 |
| Wildtype | 0,16 | 0,01 | 0,01 | 0,05 | 0,08 |

**Example E: Activity measurement of recombinant mutated forms of cod trypsin I**

Materials & Methods

*Expression of recombinant polypeptides*

Polypeptides corresponding to the wildtype amino acid sequence of trypsin I from Atlantic cod and thirty-eight mutated versions thereof were produced using the methods described in Example C.

*Activation*

Activation of recombinant enzymes (approximately 0.01 mg/ml) was achieved by adding wild type trypsin (0.2 U/ml) at room temperature and incubate for 24 hours. The mixture was made in 20mM Tris-HCl, 1 mM CaCl2, 50% glycerol, pH 8.0 to a final volume of 200μl.

*Activity assay to determine kinetic constants*

The substrate (Gly-Pro-Arg) was used at concentrations 0.005-0.15 mM in assay buffer containing 1% DMSO. 245 μL of substrate solutions were pipetted into a 96-well plate. The reaction was started by adding 5 μL of the sample mixture (above) and monitored at 410 nm in a SpectraMax plate reader. Kinetic measurement was performed every minute of a continuous 15-min run.

Results

The results are shown in Table 5 below.

**Table 5**

| Sample | Parameter | Value | Relative to WT-Trp |
|---|---|---|---|
| WT-Trp | Vmax (Kcat) | 0,05372 | 100 |
| (pu rified) | Km | 0,00125 | 100 |
| | Vmax /Km | 43,07 | 100 |
| | | | |
| EZA-001 | Vmax | 0,05309 | 99 |
| | Km | 0,00087 | 70 |
| | Vmax /Km | 61,10 | 142 |
| | | | |
| EZA-002 | Vmax | 0,05292 | 99 |
| | Km | 0,00110 | 88 |
| | Vmax /Km | 48,09 | 112 |
| | | | |
| EZA-003 | Vmax | 0,05162 | 96 |
| | Km | 0,00050 | 40 |
| | Vmax /Km | 104,07 | 242 |
| | | | |
| EZA-004 | Vmax | 0,04380 | 82 |
| | Km | 0,00123 | 99 |
| | Vmax /Km | 35,47 | 82 |
| | | | |
| EZA-005 | Vmax | 0,05162 | 96 |
| | Km | 0,00094 | 75 |
| | Vmax /Km | 54,95 | 128 |
| | | | |
| EZA-006 | Vmax | 0,05289 | 98 |
| | Km | 0,00095 | 76 |
| | Vmax /Km | 55,81 | 130 |
| | | | |
| EZA-007 | Vmax | 0,05313 | 99 |
| | Km | 0,00114 | 91 |
| | Vmax /Km | 46,72 | 108 |
| | | | |
| EZA-008 | Vmax | 0,05084 | 95 |
| | Km | 0,00083 | 67 |
| | Vmax /Km | 60,90 | 141 |
| | | | |
| EZA-009 | Vmax | 0,05287 | 98 |
| | Km | 0,00087 | 70 |

(continued)

| Sample | Parameter | Value | Relative to WT-Trp |
|---|---|---|---|
| | Vmax /Km | 60,98 | 142 |
| | | | |
| EZA-010 | Vmax | 0,05046 | 94 |
| | Km | 0,00085 | 68 |
| | Vmax /Km | 59,44 | 138 |
| | | | |
| EZA-011 | Vmax | 0,05045 | 94 |
| | Km | 0,00077 | 62 |
| | Vmax /Km | 65,55 | 152 |
| | | | |
| EZA-012 | Vmax | 0,04208 | 78 |
| | Km | 0,00101 | 81 |
| | Vmax /Km | 41,74302 | 97 |
| | | | |
| EZA-013 | Vmax | 0,05006 | 93 |
| | Km | 0,00068 | 55 |
| | Vmax /Km | 73,65 | 171 |
| | | | |
| EZA-014 | Vmax | 0,05177 | 96 |
| | Km | 0,00083 | 66 |
| | Vmax /Km | 62,64 | 145 |
| | | | |
| EZA-015 | Vmax | 0,05060 | 94 |
| | Km | 0,00085 | 68 |
| | Vmax /Km | 59,36 | 138 |
| | | | |
| EZA-016 | Vmax | 0,05378 | 100 |
| | Km | 0,00103 | 83 |
| | Vmax /Km | 51,99 | 121 |
| | | | |
| EZA-017 | Vmax | 0,05457 | 102 |
| | Km | 0,00104 | 83 |
| | Vmax /Km | 52,53124 | 122 |
| | | | |
| EZA-018 | Vmax | 0,05962 | 111 |
| | Km | 0,00198 | 159 |
| | Vmax /Km | 30,04 | 70 |
| | | | |

(continued)

| Sample | Parameter | Value | Relative to WT-Trp |
|---|---|---|---|
| EZA-019 | Vmax | 0,05408 | 101 |
|  | Km | 0,00115 | 92 |
|  | Vmax /Km | 47,21 | 110 |
|  |  |  |  |
| EZA-020 | Vmax | 0,04421 | 82 |
|  | Km | 0,00095 | 76 |
|  | Vmax /Km | 46,77 | 109 |
|  |  |  |  |
| EZA-021 | Vmax | 0,05309 | 99 |
|  | Km | 0,00128 | 103 |
|  | Vmax /Km | 41,41 | 96 |
|  |  |  |  |
| EZA-022 | Vmax | 0,05436 | 101 |
|  | Km | 0,00119 | 95 |
|  | Vmax /Km | 45,85 | 106 |
|  |  |  |  |
| EZA-023 | Vmax | 0,05470 | 102 |
|  | Km | 0,00137 | 110 |
|  | Vmax /Km | 40,06 | 93 |
|  |  |  |  |
| EZA-024 | Vmax | 0,05120 | 95 |
|  | Km | 0,00098 | 78 |
|  | Vmax /Km | 52,36 | 122 |
|  |  |  |  |
| EZA-025 | Vmax | 0,05145 | 96 |
|  | Km | 0,00090 | 72 |
|  | Vmax /Km | 57,43 | 133 |
|  |  |  |  |
| EZA-026 | Vmax | 0,05042 | 94 |
|  | Km | 0,00084 | 68 |
|  | Vmax /Km | 59,70 | 139 |
|  |  |  |  |
| EZA-027 | Vmax | 0,05195 | 97 |
|  | Km | 0,00094 | 76 |
|  | Vmax /Km | 55,01 | 128 |
|  |  |  |  |
| EZA-028 | Vmax | 0,04167 | 78 |
|  | Km | 0,00076 | 61 |

(continued)

| Sample | Parameter | Value | Relative to WT-Trp |
|--------|-----------|-------|--------------------|
|  | Vmax /Km | 54,60 | 127 |
|  |  |  |  |
| EZA-029 | Vmax | 0,05058 | 94 |
|  | Km | 0,00091 | 73 |
|  | Vmax /Km | 55,40 | 129 |
|  |  |  |  |
| EZA-030 | Vmax | 0,05109 | 95 |
|  | Km | 0,00080 | 65 |
|  | Vmax /Km | 63,47 | 147 |
|  |  |  |  |
| EZA-031 | Vmax | 0,05174 | 96 |
|  | Km | 0,00103 | 83 |
|  | Vmax /Km | 50,07 | 116 |
|  |  |  |  |
| EZA-032 | Vmax | 0,06226 | 116 |
|  | Km | 0,00246 | 197 |
|  | Vmax /Km | 25,31 | 59 |
|  |  |  |  |
| EZA-033 | Vmax | 0,05942 | 111 |
|  | Km | 0,00166 | 133 |
|  | Vmax /Km | 35,80 | 83 |
|  |  |  |  |
| EZA-034 | Vmax | 0,05672 | 106 |
|  | Km | 0,00144 | 116 |
|  | Vmax /Km | 39,29 | 91 |
|  |  |  |  |
| EZA-035 | Vmax | 0,05807 | 108 |
|  | Km | 0,00162 | 130 |
|  | Vmax /Km | 35,79 | 83 |
|  |  |  |  |
| EZA-036 | Vmax | 0,04887 | 91 |
|  | Km | 0,00210 | 168 |
|  | Vmax /Km | 23,28 | 54 |
|  |  |  |  |
| EZA-037 | Vmax | 0,05754 | 107 |
|  | Km | 0,00172 | 138 |
|  | Vmax /Km | 33,54 | 78 |
|  |  |  |  |

(continued)

| Sample | Parameter | Value | Relative to WT-Trp |
|---|---|---|---|
| EZA-038 | Vmax | 0,05786 | 108 |
| | Km | 0,00157 | 126 |
| | Vmax /Km | 36,74 | 85 |

**Subsequently a plurality of preferred embodiments of the invention are addressed. These embodiments are:**

1. A polypeptide having protease activity for use in the treatment or prevention of microbial infections in a subject with or susceptible to an immunodeficiency.

2. A polypeptide according to Embodiment 1 wherein the protease is selected from the group consisting of serine proteases, threonine proteases, cysteine proteases, aspartate proteases, glutamic acid proteases and metalloproteases.

3. A polypeptide according to Embodiment 2 wherein the protease is a serine protease.

4. A polypeptide according to Embodiment 3 wherein the protease is a trypsin or chymotrypsin.

5. A polypeptide according to any one of the preceding embodiments wherein the immunodeficiency is a secondary or acquired immunodeficiency.

6. A polypeptide according to Embodiment 5 wherein the subject is receiving treatment with an immunosuppressant therapy.

7. A polypeptide according to Embodiment 6 wherein the immunosuppressant therapy is selected from the group consisting of glucocorticoids, cytostatics, antibodies, drugs acting on immunophilins, interferons, opioids, TNF-binding proteins, mycophenolate and radiation therapy.

8. A polypeptide according to any one of Embodiments 1 to 4 wherein the immunodeficiency is naturally-occurring.

9. A polypeptide according to any one of the preceding embodiments wherein the immunodeficiency is due to a primary immunodeficiency, a cancer (such as leukemia, lymphoma, multiple myeloma), chronic infection (such as acquired immunodeficiency syndrome or AIDS), malnutrition and/or aging.

10. A polypeptide according to Embodiment 9 wherein the subject has a primary immunodeficiency.

11. A polypeptide according to any one of the preceding embodiments wherein the patient has a primary immunodeficiency selected from the group consisting of the indications listed in Tables I to VIII.

12. A polypeptide according to any one of the preceding embodiments for use in the treatment or prevention of secondary infections of the mouth and/or pharynx.

13. A polypeptide according to any one of the preceding embodiments for use in the treatment or prevention of rhinorrhea and/or fungal infection of the oral cavity and/or gum sores.

14. A polypeptide according to any one of the preceding embodiments wherein the microbial infection is selected from the group consisting of bacterial infections, viral infections, fungal infections and yeast infections.

15. A polypeptide according to any one of the preceding embodiments wherein the polypeptide is for use in combination with one or more additional anti-microbial treatments.

16. A polypeptide according to any one of the preceding embodiments wherein the subject is human.

17. A polypeptide according to any one of the preceding embodiments wherein the polypeptide is naturally occurring.

18. A polypeptide according to any one of Embodiments 1 to 16 wherein the polypeptide is non-naturally occurring.

19. A polypeptide according to any one of the preceding embodiments the polypeptide comprises or consists of an amino acid sequence of SEQ ID NO: 1:

IVGGYECTKHSQAHQVSLNSGYHFCGGSLVSKDWVVSAAHCYKSVLRVRLGEHHIRVN

EGTEQYISSSSVIRHPNYSSYNINNDIMLIKLTKPATLNQYVHAVALPTECAADATMCTVS

GWGNTMSSVADGDKLQCLSLPILSHADCANSYPGMITQSMFCAGYLEGGKDSCQGDS

GGPVVCNGVLQGVVSWGYGCAERDHPGVYAKVCVLSGWVRDTMANY

[SEQ ID NO: 1]

or a fragment, variant, derivative or fusion thereof (or a fusion of said fragment, variant or derivative) which retains the trypsin activity of said amino acid sequence.

20. A polypeptide according to Embodiment 19 wherein the polypeptide comprises or consists of an amino acid sequence according to SEQ ID NO: 1.

21. A polypeptide according to Embodiment 20 wherein the polypeptide is purified from cod.

22. A polypeptide according to any one of Embodiments 1 to 19 wherein the polypeptide comprises or consists of a fragment of the amino acid sequence according to SEQ ID NO: 1.

23. A polypeptide according to Embodiment 22 wherein the fragment comprises or consists of at least 15 contiguous amino acid of SEQ ID NO: 1, for example at least 16, 17, 18, 19, 20, 30, 40, 50, 60, 70, 80, 90, 100, 110, 120, 130, 140, 150, 160, 170, 180, 190, 200, 210, 220, 230 or 240 contiguous amino acid of SEQ ID NO: 1.

24. A polypeptide according to Embodiment 22 or 23 wherein the fragment comprises or consists of amino acid residues 61 to 77 of SEQ ID NO: 1.

25. A polypeptide according to Embodiment 22 or 23 wherein the fragment comprises or consists of amino acid residues 225 to 241 of SEQ ID NO: 1.

26. A polypeptide according to any one of Embodiments 1 to 19 wherein the polypeptide comprises or consists of a variant of the amino acid sequence according to SEQ ID NO: 1.

27. A polypeptide according to Embodiment 26 wherein the variant is a non-naturally occurring variant.

28. A polypeptide according to Embodiment 26 or 27 wherein the variant has an amino acid sequence which has at least 50% identity with the amino acid sequence according to SEQ ID NO: 1 or a fragment thereof, for example at least 55%, 60%, 65%, 70%, 75%, 80%, 90%, 95%, 96%, 97%, 98% or at least 99% identity.

29. A polypeptide according to any one of Embodiments 26 to 28 wherein the polypeptide having protease activity is a variant of SEQ ID NO:1, comprising one or more mutated amino acids selected from the group consisting of amino acid positions:
E21, H25, H29, V47, K49, D50, L63, H71, H72, R74, N76, T79, Y82, S85, S87, S89, N98, I99, V121, M135, V138, M145, V148, D150, K154, L160, M175, S179, A183, L185, V212, Y217, P225, A229, V233, L234, V238, N240, Y241 and/or M242
or a fragment thereof which exhibits an antimicrobial activity.

30. A polypeptide according to Embodiment 29 wherein the polypeptide having protease activity is a variant of SEQ ID NO:1, comprising one or more mutated amino acids selected from the group consisting of:
E21T, H25Y, H29(Y/N), V47I, K49E, D50Q, L63I, H71D, H72N, R74(K/E), N76(T/L), T79(S/N), Y82F, S85A, S87(K/R), S89R, N98T, I99L, V121I, M135Q, V138I, M145(T/L/V/E/K), V148G, D150S, K154(T/V), L160(I/A), M175(K/Q), S179N, A183V, L185G, V212I, Y217(D/H/S), P225Y, A229V, V233N, L234Y, V238I, N240S, Y241N and/or M242I
or a fragment thereof which exhibits an antimicrobial activity.

31. A polypeptide according to any one of Embodiments 26 to 30 wherein the polypeptide having protease activity is selected from the group of polypeptides in Table 3.

32. A polypeptide according to any one of Embodiments 26 to 31 wherein the polypeptide having protease activity comprises or consists of an amino acid sequence of SEQ ID NO: 2 or 3, or a fragment thereof which exhibits an antimicrobial activity.

33. A polypeptide according to any one of the preceding embodiments wherein the polypeptide comprises or consists of a fusion protein.

34. A polypeptide according to any one of the preceding embodiments wherein the polypeptide, or fragment, variant, fusion or derivative thereof, comprises or consists of L-amino acids.

35. A polypeptide according to any one of the preceding embodiments wherein the polypeptide, or fragment, variant, fusion or derivative thereof, comprises one or more amino acids that are modified or derivatised.

36. A polypeptide according to Embodiment 35 wherein the one or more amino acids are modified or derivatised by PEGylation, amidation, esterification, acylation, acetylation and/or alkylation.

37. A polypeptide according to any one of the preceding embodiments wherein the polypeptide is between 10 and 30 amino acids in length, for example between 10 and 20, 12 and 18, 12 and 16, or 15 and 20 amino acids in length.

38. A polypeptide according to any one of the preceding embodiments wherein the polypeptide is between 150 and 250 amino acids in length, for example between 200 and 250, 210 and 240, 220 and 230, or 220 and 225 amino acids in length.

39. A polypeptide according to any one of the preceding embodiments wherein the polypeptide is linear.

40. A polypeptide according to any one of the preceding embodiments wherein the polypeptide is a recombinant polypeptide.

41. A polypeptide according to any one of the preceding embodiments wherein the polypeptide is provided in a form suitable for delivery to the mucosa of the mouth and/or pharynx.

42. A polypeptide according to any one of the preceding embodiments wherein the polypeptide is provided in a mouth spray, nasal spray, lozenge, pastille, chewing gum or liquid.

43. A polypeptide according to any one of the preceding embodiments wherein the polypeptide is provided in a mouth spray.

44. Use of a polypeptide as defined in any one of Embodiments 1 to 39 in the preparation of a medicament for the treatment or prevention of microbial infections in a subject with or susceptible to immunodeficiency.

45. The use according to Embodiment 44 wherein the polypeptide is a trypsin or chymotrypsin.

46. The use according to Embodiment 44 or 45 wherein the polypeptide comprises or consists of an amino acid sequence of SEQ ID NO: 1 or a fragment, variant, derivative or fusion thereof (or a fusion of said fragment, variant or derivative) which retains the trypsin activity of said amino acid sequence.

47. The use according to any one of Embodiments 44 to 46 wherein the polypeptide is provided in a mouth spray, nasal spray, lozenge, pastille, chewing gum or liquid.

48. The use according to any one of Embodiments 44 to 47 wherein the subject has a primary immunodeficiency.

49. A method for the treatment or prevention of microbial infections in a subject with or susceptible to immunodeficiency, the method comprising administering to the subject a therapeutically-effective amount of a polypeptide as defined in any one of Embodiments 1 to 39.

50. The method according to Embodiment 49 wherein the polypeptide is a trypsin or chymotrypsin.

51. The method according to Embodiment 49 or 50 wherein the polypeptide comprises or consists of an amino acid sequence of SEQ ID NO: 1 or a fragment, variant, derivative or fusion thereof (or a fusion of said fragment, variant or derivative) which retains the trypsin activity of said amino acid sequence.

52. The method according to any one of Embodiments 49 to 51 wherein the polypeptide is administered in a mouth spray, nasal spray, lozenge, pastille, chewing gum or liquid.

53. The method according to any one of Embodiments 49 to 52 wherein the subject has a primary immunodeficiency.

54. A polypeptide for use in the treatment or prevention of microbial infections in a patient with primary immunodeficiency as described herein with reference to the description.

55. Use of a polypeptide in the preparation of a medicament for the treatment or prevention of microbial infections in a patient as described herein with reference to the description.

56. A method for the treatment or prevention of microbial infections in a patient with primary immunodeficiency with reference to the description.

## Claims

1. A polypeptide having protease activity for use in the treatment or prevention of microbial infections in a subject with or susceptible to an immunodeficiency.

2. A polypeptide according to Claim 1 wherein the protease is selected from the group consisting of serine proteases, threonine proteases, cysteine proteases, aspartate proteases, glutamic acid proteases and metalloproteases.

3. A polypeptide according to Claim 2 wherein the protease is a trypsin or chymotrypsin.

4. A polypeptide according to any one of the preceding claims wherein the immunodeficiency is a secondary or acquired immunodeficiency.

5. A polypeptide according to any one of Claims 1 to 3 wherein the subject has a primary immunodeficiency.

6. A polypeptide according to any one of the preceding claims wherein the patient has a primary immunodeficiency selected from the group consisting of the indications listed in Tables I to VIII.

7. A polypeptide according to any one of the preceding claims for use in the treatment or prevention of a microbial infection selected from the group consisting of:
secondary infections of the mouth and/or pharynx, rhinorrhea, fungal infection of the oral cavity and/or gum sores, bacterial infections, viral infections, fungal infections and yeast infections.

8. A polypeptide according to any one of the preceding claims wherein the polypeptide comprises or consists of an amino acid sequence of SEQ ID NO: 1:

IVGGYECTKHSQAHQVSLNSGYHFCGGSLVSKDWVVSAAHCYKSVLRVRLGEHHIRVN

EGTEQYISSSSVIRHPNYSSYNINNDIMLIKLTKPATLNQYVHAVALPTECAADATMCTVS

GWGNTMSSVADGDKLQCLSLPILSHADCANSYPGMITQSMFCAGYLEGGKDSCQGDS

GGPVVCNGVLQGVVSWGYGCAERDHPGVYAKVCVLSGWVRDTMANY

# [SEQ ID NO: 1]

or a fragment, variant, derivative or fusion thereof (or a fusion of said fragment, variant or derivative) which retains the trypsin activity of said amino acid sequence.

9. A polypeptide according to Claim 11 wherein the polypeptide comprises or consists of an amino acid sequence according to SEQ ID NO: 1.

10. A polypeptide according to any one of Claims 1 to 8 wherein the polypeptide comprises or consists of a fragment of the amino acid sequence according to SEQ ID NO: 1.

11. A polypeptide according to any one of Claims 1 to 8 wherein the polypeptide comprises or consists of a variant of the amino acid sequence according to SEQ ID NO: 1.

12. A polypeptide according to Claim 11 wherein the variant has an amino acid sequence which has at least 50% identity with the amino acid sequence according to SEQ ID NO: 1 or a fragment thereof, for example at least 55%, 60%, 65%, 70%, 75%, 80%, 90%, 95%, 96%, 97%, 98% or at least 99% identity.

13. A polypeptide according to Claim 11 or 12 wherein the polypeptide having protease activity is a variant of SEQ ID NO:1, comprising one or more mutated amino acids selected from the group consisting of amino acid positions:
E21, H25, H29, V47, K49, D50, L63, H71, H72, R74, N76, T79, Y82, S85, S87, S89, N98, I99, V121, M135, V138, M145, V148, D150, K154, L160, M175, S179, A183, L185, V212, Y217, P225, A229, V233, L234, V238, N240, Y241 and/or M242
or a fragment thereof which exhibits an antimicrobial activity.

14. A polypeptide according to any one of Claims 11 to 13 wherein the polypeptide having protease activity is selected from the group of polypeptides in Table 3.

15. A polypeptide according to any one of the preceding claims wherein the polypeptide is provided in a form suitable for delivery to the mucosa of the mouth and/or pharynx.

# FIGURE 1

(A)

(B)

# FIGURE 2

(A)

(B)

Europäisches
Patentamt

European
Patent Office

Office européen
des brevets

# EUROPEAN SEARCH REPORT

Application Number

EP 21 18 0866

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/78332 A2 (BJARNASON JON BRAGI [IS]) 28 December 2000 (2000-12-28) * page 7, lines 13-15 page 8, lines 24-25; claims 1,4,5-12, 16, 32-38; example 2 * | 1-15 | INV. A61K38/48 A61P31/00 A61P31/04 A61P31/10 A61P31/12 A61P11/00 |
| X | WO 2011/050135 A1 (CUREMARK LLC [US]; HEIL MATTHEW [US]; FALLON JAMES J [US]; FALLON JOAN) 28 April 2011 (2011-04-28) * paragraphs [0078], [0079], [0082], [0083]; claims 1-7 * | 1-4,7,8, 11,14,15 | |
| X | WO 02/06460 A2 (JENSENIUS JENS CHRISTIAN [DK]; THIEL STEFFEN [DK]) 24 January 2002 (2002-01-24) * claims 1,22-26, 30,32 * | 1,2,4,7, 8,11,14, 15 | |
| A | ÁGÚSTA GUDMUNDSDÓTTIR ET AL: "Potential Use of Atlantic Cod Trypsin in Biomedicine", BIOMED RESEARCH INTERNATIONAL, vol. 54, no. 8, 1 January 2013 (2013-01-01), pages 16-11, XP055192029, ISSN: 2314-6133, DOI: 10.1097/WON.0b013e3181bfdf83 * abstract * | 1-15 | |

TECHNICAL FIELDS
SEARCHED     (IPC)

A61K
A61P
C12N

The present search report has been drawn up for all claims

| Place of search | Date of completion of the search | Examiner |
|---|---|---|
| Munich | 23 November 2021 | Habedanck, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 0866

23-11-2021

| Patent document cited in search report | | | Publication date | Patent family member(s) | | | Publication date |
|---|---|---|---|---|---|---|---|
| WO 0078332 | A2 | | 28-12-2000 | AT | 278417 | T | 15-10-2004 |
| | | | | AU | 779700 | B2 | 10-02-2005 |
| | | | | CA | 2377357 | A1 | 28-12-2000 |
| | | | | CN | 1356907 | A | 03-07-2002 |
| | | | | DE | 60014659 | T2 | 02-03-2006 |
| | | | | DK | 1202743 | T3 | 14-02-2005 |
| | | | | EP | 1202743 | A2 | 08-05-2002 |
| | | | | ES | 2231200 | T3 | 16-05-2005 |
| | | | | IS | 6194 | A | 10-12-2001 |
| | | | | JP | 2003502071 | A | 21-01-2003 |
| | | | | KR | 20020025082 | A | 03-04-2002 |
| | | | | MX | PA01013246 | A | 20-08-2003 |
| | | | | NO | 327283 | B1 | 02-06-2009 |
| | | | | NZ | 516632 | A | 30-04-2004 |
| | | | | PL | 352318 | A1 | 11-08-2003 |
| | | | | PT | 1202743 | E | 28-02-2005 |
| | | | | US | 2002141987 | A1 | 03-10-2002 |
| | | | | WO | 0078332 | A2 | 28-12-2000 |
| WO 2011050135 | A1 | | 28-04-2011 | US | 2012258149 | A1 | 11-10-2012 |
| | | | | US | 2017202934 | A1 | 20-07-2017 |
| | | | | US | 2020282030 | A1 | 10-09-2020 |
| | | | | WO | 2011050135 | A1 | 28-04-2011 |
| WO 0206460 | A2 | | 24-01-2002 | AT | 434661 | T | 15-07-2009 |
| | | | | AU | 7958601 | A | 30-01-2002 |
| | | | | CA | 2415751 | A1 | 24-01-2002 |
| | | | | CA | 2887012 | A1 | 24-01-2002 |
| | | | | CN | 1449440 | A | 15-10-2003 |
| | | | | DK | 1303591 | T3 | 02-11-2009 |
| | | | | EP | 1303591 | A2 | 23-04-2003 |
| | | | | JP | 5226169 | B2 | 03-07-2013 |
| | | | | JP | 2004504027 | A | 12-02-2004 |
| | | | | NZ | 523900 | A | 27-02-2004 |
| | | | | US | 2004038297 | A1 | 26-02-2004 |
| | | | | WO | 0206460 | A2 | 24-01-2002 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1202743 B
- US 4235871 A
- US 5851451 A
- EP 0213303 A

**Non-patent literature cited in the description**

- **RIVAT et al.** *Hum. Gene Ther.,* 2012, vol. 23 (7), 668-675
- **ÁSGEIRSSON et al.** *Eur. J. Biochem.,* 1989, vol. 180, 85-94
- **ÁSGEIRSSON ; BJARNASON.** *Comp. Biochem. Physiol. B,* 1991, vol. 998, 327-335
- **SAMBROOK ; RUSSELL.** Molecular Cloning: a Laboratory Manual. Cold Spring Harbor Laboratory Press, 2000
- **THOMPSON et al.** *Nuc. Acid Res.,* 1994, vol. 22, 4673-4680
- **MEZIERE et al.** *J. Immunol.,* 1997, vol. 159, 3230-3237
- **VEBER et al.** *Proc. Natl. Acad. Sci. USA,* 1978, vol. 75, 2636
- **THURSELL et al.** *Biochem. Biophys. Res. Comm.,* 1983, vol. 111, 166
- **SAMBROOK ; RUSSELL.** Molecular Cloning, A Laboratory Manual. Cold Spring Harbor, 2000
- Remington's Pharmaceutical Sciences. Mack Publishing Company, 1990
- Pharmaceutical Excipients. Pharmaceutical Press, 2000

SEQUENCE LISTING

<110> Enzymatica AB

<120> PID

<130> P57543EPdiv1

<160> 5

<170> BiSSAP 1.3.6

<210> 1
<211> 222
<212> PRT
<213> Gadus morhua

<220>
<223> Trypsin I

<400> 1
Ile Val Gly Gly Tyr Glu Cys Thr Lys His Ser Gln Ala His Gln Val
1               5                   10                  15
Ser Leu Asn Ser Gly Tyr His Phe Cys Gly Gly Ser Leu Val Ser Lys
            20                  25                  30
Asp Trp Val Val Ser Ala Ala His Cys Tyr Lys Ser Val Leu Arg Val
        35                  40                  45
Arg Leu Gly Glu His His Ile Arg Val Asn Glu Gly Thr Glu Gln Tyr
    50                  55                  60
Ile Ser Ser Ser Ser Val Ile Arg His Pro Asn Tyr Ser Ser Tyr Asn
65                  70                  75                  80
Ile Asn Asn Asp Ile Met Leu Ile Lys Leu Thr Lys Pro Ala Thr Leu
                85                  90                  95
Asn Gln Tyr Val His Ala Val Ala Leu Pro Thr Glu Cys Ala Ala Asp
            100                 105                 110
Ala Thr Met Cys Thr Val Ser Gly Trp Gly Asn Thr Met Ser Ser Val
        115                 120                 125
Ala Asp Gly Asp Lys Leu Gln Cys Leu Ser Leu Pro Ile Leu Ser His
    130                 135                 140
Ala Asp Cys Ala Asn Ser Tyr Pro Gly Met Ile Thr Gln Ser Met Phe
145                 150                 155                 160
Cys Ala Gly Tyr Leu Glu Gly Gly Lys Asp Ser Cys Gln Gly Asp Ser
                165                 170                 175
Gly Gly Pro Val Val Cys Asn Gly Val Leu Gln Gly Val Val Ser Trp
                180                 185                 190
Gly Tyr Gly Cys Ala Glu Arg Asp His Pro Gly Val Tyr Ala Lys Val
            195                 200                 205
Cys Val Leu Ser Gly Trp Val Arg Asp Thr Met Ala Asn Tyr
    210                 215                 220

<210> 2
<211> 221
<212> PRT
<213> Gadus morhua

<220>
<223> H25N

<400> 2
Ile Val Gly Gly Tyr Glu Cys Thr Lys Asn Ser Gln Ala His Gln Val
1               5                   10                  15

31

```
Ser Leu Asn Ser Gly Tyr His Phe Cys Gly Gly Ser Leu Val Ser Lys
            20                  25                  30
Asp Trp Val Val Ser Ala Ala His Cys Tyr Lys Ser Val Leu Arg Val
        35                  40                  45
Arg Leu Gly Glu His His Ile Arg Val Asn Glu Gly Thr Glu Gln Tyr
    50                  55                  60
Ile Ser Ser Ser Ser Val Ile Arg His Pro Asn Tyr Ser Ser Tyr Asn
65                  70                  75                  80
Ile Asn Asn Asp Ile Met Leu Ile Lys Leu Thr Lys Pro Ala Thr Leu
                85                  90                  95
Asn Gln Tyr Val His Ala Val Ala Leu Pro Thr Glu Cys Ala Ala Asp
            100                 105                 110
Ala Met Cys Thr Val Ser Gly Trp Gly Asn Thr Met Ser Ser Val Ala
            115                 120                 125
Asp Gly Asp Lys Leu Gln Cys Leu Ser Leu Pro Ile Leu Ser His Ala
    130                 135                 140
Asp Cys Ala Asn Ser Tyr Pro Gly Met Ile Thr Gln Ser Met Phe Cys
145                 150                 155                 160
Ala Gly Tyr Leu Glu Gly Gly Lys Asp Ser Cys Gln Gly Asp Ser Gly
            165                 170                 175
Gly Pro Val Val Cys Asn Gly Val Leu Gln Gly Val Val Ser Trp Gly
            180                 185                 190
Tyr Gly Cys Ala Glu Arg Asp His Pro Gly Val Tyr Ala Lys Val Cys
            195                 200                 205
Val Leu Ser Gly Trp Val Arg Asp Thr Met Ala Asn Tyr
            210                 215                 220


<210> 3
<211> 221
<212> PRT
<213> Gadus morhua


<220>
<223> L160I

<400> 3
Ile Val Gly Gly Tyr Glu Cys Thr Lys His Ser Gln Ala His Gln Val
1                   5                   10                  15
Ser Leu Asn Ser Gly Tyr His Phe Cys Gly Gly Ser Leu Val Ser Lys
            20                  25                  30
Asp Trp Val Val Ser Ala Ala His Cys Tyr Lys Ser Val Leu Arg Val
        35                  40                  45
Arg Leu Gly Glu His His Ile Arg Val Asn Glu Gly Thr Glu Gln Tyr
    50                  55                  60
Ile Ser Ser Ser Ser Val Ile Arg His Pro Asn Tyr Ser Ser Tyr Asn
65                  70                  75                  80
Ile Asn Asn Asp Ile Met Leu Ile Lys Leu Thr Lys Pro Ala Thr Leu
                85                  90                  95
Asn Gln Tyr Val His Ala Val Ala Leu Pro Thr Glu Cys Ala Ala Asp
            100                 105                 110
Ala Met Cys Thr Val Ser Gly Trp Gly Asn Thr Met Ser Ser Val Ala
            115                 120                 125
Asp Gly Asp Lys Leu Gln Cys Leu Ser Ile Pro Ile Leu Ser His Ala
    130                 135                 140
Asp Cys Ala Asn Ser Tyr Pro Gly Met Ile Thr Gln Ser Met Phe Cys
145                 150                 155                 160
Ala Gly Tyr Leu Glu Gly Gly Lys Asp Ser Cys Gln Gly Asp Ser Gly
            165                 170                 175
Gly Pro Val Val Cys Asn Gly Val Leu Gln Gly Val Val Ser Trp Gly
            180                 185                 190
Tyr Gly Cys Ala Glu Arg Asp His Pro Gly Val Tyr Ala Lys Val Cys
            195                 200                 205
```

```
Val Leu Ser Gly Trp Val Arg Asp Thr Met Ala Asn Tyr
    210                 215                 220


<210> 4
<211> 678
<212> DNA
<213> Gadus morhua


<220>
<223> WT Trypsin I in pUC57

<400> 4
gaagaagata aaatcgttgg cggctatgaa tgcacgaaac actcgcaggc acaccaggtc      60

tcactgaaca gcggttacca cttttgcggc ggtagtctgg ttagcaaaga ttgggttgtt     120

agtgcggccc attgctataa aagcgtgctg cgtgttcgcc tgggcgaaca tcacattcgt     180

gtgaatgaag gcaccgaaca gtacattagc tctagtagcg ttatccgcca tccgaactac     240

tctagttaca acatcaacaa cgatatcatg ctgatcaaac tgaccaaacc ggcgacgctg     300

aaccagtatg tgcacgccgt tgcactgccg accgaatgcg cagcggatgc aaccatgtgt     360

accgtgagcg gctggggtaa tacgatgagc tctgttgcgg atggcgataa actgcagtgc     420

ctgtctctgc cgattctgag tcatgcggat tgtgccaact cttatccggg catgatcacg     480

cagagcatgt tttgcgccgg ttacctggaa ggcggtaaag atagctgcca gggtgattct     540

ggcggtccgg tggtttgtaa cggcgttctg cagggtgtgg ttagctgggg ctacggttgt     600

gcagaacgtg atcacccggg tgtctatgct aaagtctgtg tgctgtcggg ctgggtccgt     660

gatacgatgg cgaactat                                                   678


<210> 5
<211> 5
<212> PRT
<213> Gadus morhua


<220>
<223> Activation peptide

<400> 5
Met Glu Glu Asp Lys
1               5
```